(19) Europäisches Patentamt
European Patent Office
Office européen des brevets

(11) EP 0 672 660 B1

(12) EUROPÄISCHE PATENTSCHRIFT

(45) Veröffentlichungstag und Bekanntmachung des Hinweises auf die Patenterteilung:
**09.11.2005 Patentblatt 2005/45**

(21) Anmeldenummer: **95101747.4**

(22) Anmeldetag: **09.02.1995**

(51) Int Cl.[7]: **C07D 235/06**, C07D 417/12, A61K 31/415, A61K 31/425, C07D 417/04, C07D 403/12, C07D 405/12, C07D 491/10, C07D 405/04, C07D 493/10

(54) **Substituierte Cyclohexanolester, ihre Verwendung zur Behandlung von Krankheiten und pharmazeutische Präparate**

Substituted cyclohexanol-esters, their use to treat illnesses and pharmaceutical preparations

Esters de cyclohexanole substitués, leur utilisation pour le traitement des maladies et préparations pharmaceutiques

(84) Benannte Vertragsstaaten:
**AT BE CH DE DK ES FR GB GR IE IT LI LU NL PT SE**

(30) Priorität: **16.02.1994 DE 4404848**

(43) Veröffentlichungstag der Anmeldung:
**20.09.1995 Patentblatt 1995/38**

(73) Patentinhaber: **Sanofi-Aventis Deutschland GmbH
65929 Frankfurt am Main (DE)**

(72) Erfinder:
- **Hemmerle, Horst, Dr.
D-64653 Lorsch (DE)**
- **Schubert, Gerrit, Dr.
D-65779 Kelkheim (DE)**
- **Below, Peter, Dr.
D-60529 Frankfurt (DE)**
- **Herling, Andreas, Dr.
D-65520 Bad Camberg (DE)**
- **Burger, Hans-Jörg, Dr.
D-65719 Hofheim (DE)**

(56) Entgegenhaltungen:
**EP-A- 0 587 087**
**EP-A- 0 587 088**
**DE-A- 4 202 184**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 672 660 B1

Printed by Jouve, 75001 PARIS (FR)

## Beschreibung

Das Krankheitsbild des Diabetes ist durch erhöhte Blutzuckerwerte gekennzeichnet. Beim insulinpflichtigen oder Typ I Diabetes ist die Ursache das Absterben der insulinproduzierenden β-Zellen des Pankreas; die Behandlung erfolgt daher durch Insulingabe (Substitutionstherapie). Der nicht-insulinabhängige oder Typ II Diabetes ist dagegen durch eine verminderte Insulinwirkung auf Muskel- und Fettgewebe (Insulinresistenz) und eine gesteigerte Glucoseproduktion der Leber gekennzeichnet. Die Ursachen dieser Stoffwechselstörungen sind noch weitgehend ungeklärt. Die etablierte Therapie mit Sulfonylharnstoffen versucht die Insulinresistenz durch Steigerung der körpereigenen Insulinfreisetzung zu kompensieren, führt jedoch nicht in allen Fällen zu einer Normalisierung des Blutzuckerspiegels und vermag das Fortschreiten der Krankheit nicht aufzuhalten; viele Typ II Diabetiker werden schließlich durch "Erschöpfung" der β-Zellen insulinpflichtig und leiden an Spätschäden wie Katarakten, Nephropathien und Angiopathien.

Neue Therapieprinzipien zur Behandlung des Typ II Diabetes sind deshalb wünschenswert.

Die Konzentration der Blutglucose im nüchternen Zustand wird durch die Glucoseproduktion der Leber bestimmt. Verschiedene Arbeitsgruppen konnten zeigen, daß die Erhöhung der Blutzuckerwerte bei Typ II Diabetes mit einer proportional erhöhten Glucoseabgabe aus der Leber korrelieren. Die von der Leber in das Blut abgegebene Glucose kann sowohl durch Abbau von Leber-Glycogen (Glycogenolyse) als auch durch Gluconeogenese gebildet werden.

Glucose-6-Phosphat ist das gemeinsame Endprodukt sowohl der Gluconeogenese als auch der Glycogenolyse. Der terminale Schritt der hepatischen Freisetzung von Glucose aus Glucose-6-Phosphat wird von der Glucose-6-Phosphatase (EC 3.1.3.9) katalysiert. Die Glucose-6-Phosphatase stellt einen im endoplasmatischen Reticulum (ER) vorkommenden Multienzym-Komplex dar. Dieser Enzym-Komplex besteht aus einer in der ER-Membran vorliegenden Glucose-6-Phosphat-Translocase, einer auf der luminalen Seite des endoplasmatischen Reticulum lokalisierten Glucose-6-Phoshatase und einer Phosphat-Translocase [für eine Übersicht siehe: Ashmore J. und Weber G., "The Role of Hepatic Glucose-6-phosphatase in the Regulation of Carbohydrate Metabolism", in Vitamins und Hormones, Vol XVII (Harris R. S., Marrian G. F., Thimann K. V., Edts.), 92 bis 132, (1959); Burchell A., Waddell I. D., "The molecular basis of the hepatic microsomal glucose-6-phosphatase system", Biochim. Biophys. Acta 1092, 129 bis 137, (1990)]. Die vorliegende umfangreiche Literatur zeigt , daß unter allen untersuchten Bedingungen, die im Tierversuch zu erhöhten Blutzuckerwerten führen, z.B. Streptozotocin, Alloxan, Cortison, Thyroid-Hormone und Hungern, die Aktivität dieses Multienzym-Komplexes ebenfalls erhöht ist. Darüber hinaus weisen zahlreiche Untersuchungen darauf hin, daß die bei Typ II Diabetikern beobachtete erhöhte Glucose-Produktion mit einer erhöhten Glucose-6-Phosphatase-Aktivität verbunden ist.

Die Bedeutung des Glucose-6-Phosphatase-Systems für eine normale Glucose-Homeostase wird ferner unterstrichen durch die hypoglykämischen Symptome von Patienten mit Glykogen-Speicherkrankheit Typ Ib, denen die Translocase-Komponente des Glucose-6-Phosphat-Systems fehlt.

Eine Verringerung der Glucose-6-Phosphatase-Aktivität durch geeignete Wirkstoffe (Inhibitoren) sollte zu einer verringerten hepatischen Glucose-Freisetzung führen. Diese Wirkstoffe sollten in der Lage sein, die Glucose-Produktion der Leber dem effektiven peripheren Verbrauch anzupassen. Die dadurch im nüchternen Zustand von Typ II Diabetikern gesenkten Blutglucose-Werte dürften darüber hinaus auch eine präventive Wirkung im Hinblick auf diabetische Spätschäden haben.

In der Literatur ist eine Reihe von unspezifischen Inhibitoren der Glucose-6-Phophatase beschrieben worden, z.B. Phlorrhizin [Soodsma, J. F., Legler, B. und Nordlie, R. C., J. Biol. Chem. 242, 1955 bis 1960, (1967)], 5,5'-Dithio-bis-2-nitro-benzoesäure [Wallin, B. K. und Arion, W. J., Biochem. Biophys. Res. Commun. 48, 694 bis 699, (1972)], 2,2'-Di-isothiocyanato-stilben und 2-Isothiocyanato-2'-acetoxy-stilben [Zoccoli, M. A. und Karnowski, M. L., J. Biol. Chem. 255, 1113 bis 1119, (1980)]. Die ersten therapeutisch verwertbaren Inhibitoren des Glucose-6-Phosphatase-Systems werden in den europäischen Patentanmeldungen Nr. 93 114 260.8 und Nr. 93 114 261.6 vorgeschlagen.

Die nachfolgend näher charakterisierten Cyclohexan-Derivate sind neue, in der chemischen und biologischen Literatur bisher nicht beschriebene Verbindungen. Wir haben nun gefunden, daß Ester von bestimmten Cyclohexanol-Derivaten, z.B. die Verbindung gemäß Beispiel 4, sehr gute Hemmer des Glucose-6-Phosphatase-Systems sind.

Die Erfindung betrifft daher Cyclohexanol-Ester der Formel I

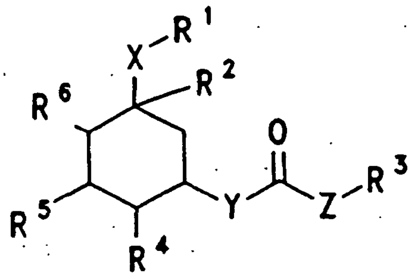

I

worin die Reste die folgende Bedeutung haben:

$R^1$ = $CONHCOR^{15}$, $CSNHR^{15}$, $CONHSO_2R^{14}$, $CSNHSO_2R^{14}$ oder $CH_2NHSO_2R^{14}$ ist oder einen Rest der folgenden Formen darstellt:

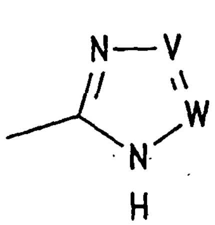 , 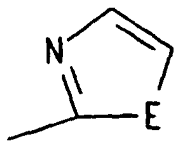 , 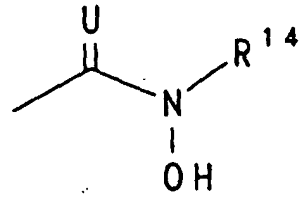 ,

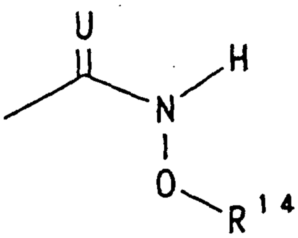 , 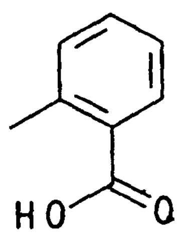 oder 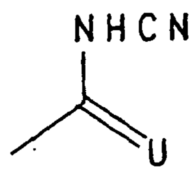

worin E = O, S oder NH, $R^{14}$ = H, U = O oder S, V = N oder CH, W = N oder CH bedeuten und aromatische Ringe einfach oder mehrfach mit F, Cl, Br, I, OH, O-$C_1$-$C_4$-Alkyl, $C_1$-$C_4$-Alkyl, $CF_3$, $NO_2$, CN, substituiert sein können, oder $R^1$ bildet gemeinsam mit $R^2$ den Ring

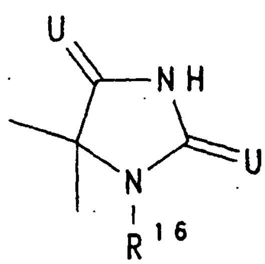

$R^2$: O-$C_1$-$C_{10}$-Alkyl$(R^{11})_n$, (n = 0, 1, 2), wobei der Alkylteil unverzweigt, verzweigt oder cyclisch ist, und $R^{11}$ mit $R^{12}$ substituiert sein kann und bei n = 2 die beiden Reste $R^{11}$ gleich oder verschieden sind, O-$C_3$-$C_{10}$-Alkenyl$(R^{11})_n$ (n = 0, 1, 2), wobei der Alkenylteil unverzweigt, verzweigt oder cyclisch ist, und ein- oder mehrfach ungesättigt ist, und $R^{11}$ mit $R^{12}$ substituiert sein kann und bei n = 2 die beiden Reste $R^{11}$ gleich oder verschieden sind, O-$C_3$-$C_{10}$-Alkinyl$(R^{11})_n$ (n = 0, 1, 2), wobei der Alkinylteil unverzweigt, verzweigt oder cyclisch ist und ein- oder mehrfach

ungesättigt ist und $R^{11}$ mit $R^{12}$ substituiert sein kann,

$R^3$, $R^{11}$ und $R^{13}$: Alkyl mit 1 bis 10 C-Atomen, Cycloalkyl mit 3 bis 8 Ring-C-Atomen, Phenyl, Naphthyl, Phenanthryl, Pyridyl, Thienyl, Furyl, Pyrimidyl, Indolyl, Imidazolyl, Cumarinyl, Phthaliminyl, Chinolyl, Piperazinyl, Tetrazolyl, Triazolyl, Oxazolyl oder deren thieno-, pyridino-, pyrimidino- oder benzoanellierte Derivate, wobei der Aromat bzw. Heteroaromat ein- oder mehrfach mit F, Cl, Br, I, OH, $-NO_2$, CN, $C_1$-$C_4$-Alkoxy, $C_1$-$C_4$-Alkyl, $NR^8R^9$, Phenyl, Benzyl, Thienyl, Furyl, Imidazolyl, Pyridyl, O-Phenyl oder O-Benzyl gleich oder verschieden substituiert sein kann, und $R^3$, $R^{11}$ und $R^{13}$ gleich oder verschieden sind;

$R^4$, $R^5$ und $R^6$: H, OH, eine durch übliche Alkoholschutzgruppen geschützte OH-Gruppe, wobei $R^4$, $R^5$ und $R^6$ gleich oder verschieden sind;

$R^8$ und $R^9$: H, $C_1$-$C_4$-Alkyl, $C_1$-$C_4$-Alkanoyl, Phenyl, das gegebenenfalls substituiert ist mit F, Cl, Br, I, OH, O-$C_1$-$C_4$-Alkyl, $CF_3$, $-NO_2$ oder CN, wobei $R^8$ und $R^9$ gleich oder verschieden sind, oder $R^8$ und $R^9$ bilden zusammen mit dem Stickstoffatom einen 4 bis 10gliedrigen, gesättigten heterocyclischen Ring, bei dem gegebenenfalls eine $CH_2$-Gruppe durch O, S oder $NR^{10}$ ersetzt sein kann, $R^{10}$: H, $C_1$-$C_4$-Alkyl, Phenyl oder Benzyl

$R^{12}$: Phenyl, Naphthyl, Phenanthryl, Pyridyl, Thienyl, Furyl, Thiazolyl, Pyrimidyl, Indolyl, Imidazolyl, Coumarinyl, Phthaliminyl, Chinolyl, Piperazinyl, Tetrazolyl, Triazolyl, Oxazolyl oder deren thieno- oder benzoanellierte Derivate, wobei der Aromat bzw. Heteroaromat ein- oder mehrfach mit F, Cl, Br, I, OH, $CF_3$, $-NO_2$, CN, $C_1$-$C_4$-Alkoxy, $C_1$-$C_4$-Alkyl, $NR^8R^9$, Phenyl, Benzyl, Thienyl, Furyl, Imidazolyl, Pyridyl, O-Phenyl oder O-Benzyl gleich oder verschieden substituiert sein kann;

$R^{14}$: Wasserstoff, $C_1$-$C_{10}$-Alkyl, Phenyl, Naphthyl, Phenanthryl, Pyridyl, Thienyl, Furyl, Thiazolyl, Pyrimidyl, Indolyl, Imidazolyl, Coumarinyl, Phthaliminyl, Chinolyl, Piperazinyl, Tetrazolyl, Triazolyl, Oxazolyl oder deren thieno- oder benzoanellierte Derivate, wobei der Aromat bzw. Heteroaromat ein- oder mehrfach mit F, Cl, Br, I, OH, $CF_3$, $-NO_2$, CN, $C_1$-$C_4$-Alkoxy, $C_1$-$C_4$-Alkyl, $NR^8R^9$, Phenyl, Benzyl, Thienyl, Furyl, Imidazolyl, Pyridyl, O-Phenyl oder O-Benzyl gleich oder verschieden substituiert sein kann oder $R^{14}$ ist ein Rest der Formel

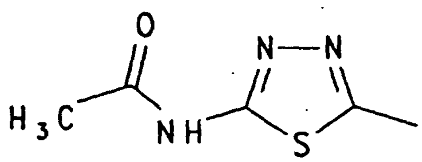

$R^{15}$: $C_3$-$C_{10}$-Alkenoyl, $C_3$-$C_{10}$-Alkenoyl($R^{12}$), $C_1$-$C_{10}$-Alkanoyl($R^{12}$), Phenyl, Naphthyl, Phenanthryl, Pyridyl, Thienyl, Furyl, Thiazolyl, Pyrimidyl, Indolyl, Imidazolyl, Coumarinyl, Phthaliminyl, Chinolyl, Piperazinyl, Tetrazolyl, Triazolyl, Oxazolyl oder deren thieno- oder benzoanellierte Derivate, wobei der Aromat bzw. Heteroaromat ein- oder mehrfach mit F, Cl, Br, I, OH, $CF_3$, $-NO_2$, CN,
$C_1$-$C_4$-Alkoxy, $C_1$-$C_4$-Alkyl, $NR^8R^9$, Phenyl, Benzyl, Thienyl, Furyl, Imidazolyl, Pyridyl, O-Phenyl oder O-Benzyl gleich oder verschieden substituiert sein kann;

$R^{16}$: $C_1$-$C_{10}$-Alkyl($R^{11}$)n, $C_3$-$C_{10}$-Alkenyl($R^{11}$)n oder $C_3$-$C_{10}$-Alkinyl($R^{11}$)n mit n jeweils null oder eins;

X: $(CH_2)_m$, (m = 0, 1, 2, 3, 4) CH = CH, C ≡ C, $CH_2OCH_2$ oder $CH_2SCH_2$.

Y: $(CH_2)_m$, (m = 0, 1, 2, 3, 4) O, S oder $NR^8$;

Z: $(CH_2)_m$, (m = 0, 1, 2, 3, 4) S, O, S-$C_1$-$C_{10}$-Alkyl, CH=CH, CH=CF, CH = CCl, CH = CBr, $CH_2$-C(O), $CH_2$-CHF, $CH_2$-CHCl, $CH_2$-CHBr, $CH_2$-CHI, $C_3$-$C_{10}$-Cycloalkylen, $C_3$-$C_{10}$-Cycloalkenylen, $COOR^7$, C=C, CH = C($C_1$-$C_4$-Alkyl) CH = C(CN), CH = C($R^{13}$) oder $NR^8$.

Bevorzugt sind Verbindungen der Formel I,
dadurch gekennzeichnet, daß die Reste in Formel I folgende Bedeutungen haben:

$R^1$: $CONHCOR^{15}$, $CSNHR^{15}$, $CONHSO_2R^{14}$, $CSNHSO_2R^{14}$ oder einen Rest der Formeln:

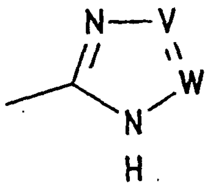 , 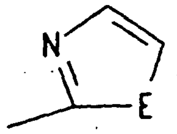 , 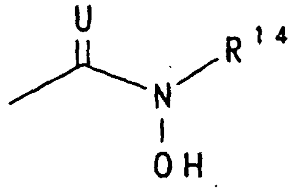 oder 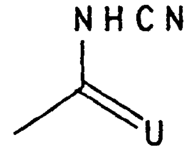

worin E = O, S oder NH, $R^{14}$ = H, U = O oder S, V = N oder CH, W = N oder CH bedeuten und aromatische Ringe einfach oder mehrfach mit F, Cl, Br, I, OH, O-$C_1$-$C_4$-Alkyl, $C_1$-$C_4$-Alkyl, $CF_3$, $NO_2$, CN, substituiert sein können oder $R^1$ bildet gemeinsam mit $R^2$ den Ring

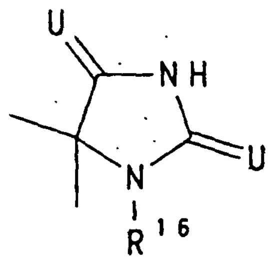

$R^2$: O-$C_1$-$C_{10}$-Alkyl($R^{11}$)$_n$, (n = 0, 1, 2), wobei der Alkylteil unverzweigt, verzweigt oder cyclisch ist, und $R^{11}$ mit $R^{12}$ substituiert sein kann und bei n = 2 die beiden Reste $R^{11}$ gleich oder verschieden sind.
O-$C_3$-$C_{10}$-Alkenyl($R^{11}$)$_n$, (n = 0, 1, 2), wobei der Alkenylteil unverzweigt, verzweigt oder cyclisch ist, sowie ein- oder mehrfach ungesättigt ist, und $R^{11}$ mit $R^{12}$ substituiert sein kann und bei n = 2 die beiden Reste $R^{11}$ gleich oder verschieden sind,
O-$C_3$-$C_{10}$-Alkinyl($R^{11}$)$_n$, (n = 0, 1, 2), wobei der Alkinylteil unverzweigt, verzweigt oder cyclisch ist, ein- oder mehrfach ungesättigt ist und $R^{11}$ mit $R^{12}$ substituiert sein kann;

X: $(CH_2)_m$, (m = 0, 1, 2, 3, 4) CH =CH, C≡C, $CH_2OCH_2$ oder $CH_2SCH_2$;

Y: $(CH_2)_m$, (m = 0, 1, 2, 3, 4) O, S oder $NR^8$;

Z: $(CH_2)_m$, (m = 0, 1, 2, 3, 4) S, O, S-$C_1$-$C_{10}$-Alkyl, (unverzweigt oder verzweigt), CH=CH, CH=CF, CH = CCl, CH=CBr, $CH_2$-C(O), $CH_2$-CHF, $CH_2$-CHCl, $CH_2$-CHBr, $CH_2$-CHI, $C_3$-$C_{10}$-Cycloalkylen, $C_3$-$C_{10}$-Cycloalkenylen, $COOR^7$, C=C, CH = C($C_1$-$C_4$-Alkyl) (unverzweigt oder verzweigt), CH = C(CN), CH = C($R^{13}$) oder $NR^8$;

$R^{16}$: $C_1$-$C_{10}$-Alkyl($R^{11}$)$_n$, $C_3$-$C_{10}$-Alkenyl($R^{11}$)$_n$, $C_3$-$C_{10}$-Alkinyl($R^{11}$)$_n$ mit n jeweils null oder 1.

Besonders bevorzugt sind Verbindungen der Formel I,
dadurch gekennzeichnet, daß die Reste in Formel I folgende Bedeutungen haben:

$R^1$: $CONHSO_2R^{14}$ oder einen Rest Rest der folgenden Formeln:

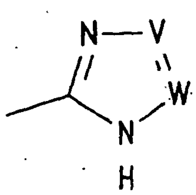 oder 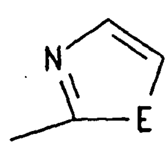

worin E = O, V = N, W = N oder $R^1$ bildet gemeinsam mit $R^2$ den Ring

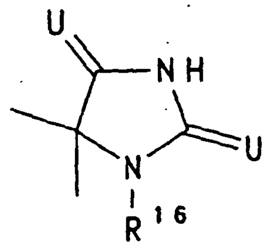

worin U Sauerstoff bedeutet,

$R^2$: O-$C_1$-$C_6$-Alkyl$(R^{11})_n$, (n = 1), wobei der Alkylteil unverzweigt, verzweigt oder cyclisch ist, O-$C_3$-$C_6$-Alkenyl $(R^{11})_n$, (n = 1), wobei der Alkenylteil unverzweigt, verzweigt oder cyclisch ist;

$R^3$, $R^{11}$ und $R^{13}$: Phenyl, mit OH oder Chlor substituiertes Phenyl, Imidazolyl, benzoanelliertes oder pyridinoanelliertes Imidazolyl, wobei $R^3$, $R^{11}$ und $R^{13}$ gleich oder verschieden sind;

$R^4$, $R^5$ und $R^6$: H oder OH, wobei $R^4$, $R^5$ und $R^6$ gleich oder verschieden sind.

$R^8$ und $R^9$: $C_1$-$C_4$-Alkyl;

$R^{14}$: $C_1$-$C_4$-Alkyl, Phenyl, Naphthyl, Thiazolyl oder dessen benzoanllierte Derivate, wobei der Aromat oder Heteroaromat mono- oder disubstituiert sein kann mit Chlor, $CF_3$, $NO_2$, $C_1$-$C_4$-Alkoxy oder $NR^8$, $R^9$, oder $R^{14}$ ist ein Rest der Formel

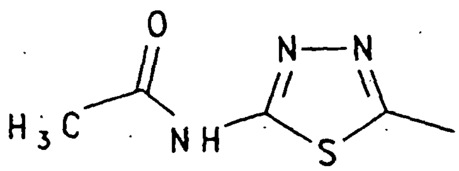

$R^{16}$: $C_1$-$C_4$-Alkyl$(R^{11})_n$ mit n = 1.

Die erfindungsgemäßen Verbindungen der Formel I können, soweit sie eine Carboxylgruppe enthalten, Salze mit anorganischen oder organischen Basen bilden. Bevorzugt sind Salze mit anorganischen Basen, besonders die physiologisch unbedenklichen Alkalimetall-Salze, vor allem Natrium- und Kaliumsalze.
Die Verbindungen der Formel I hemmen das Glucose-6-Phosphatase System der Leber in Säugetieren. Die Verbindungen eigenen sich daher als Arzneimittel. Die Erfindung betrifft daher auch Arzneimittel auf Basis der Verbindungen der Formel I, gegebenfalls in Form der physiologisch verträglichen Salze.

Die Erfindung betrifft weiterhin die Anwendung von Verbindungen der Formel I oder der Salze zur Herstellung von Arzneimitteln zur Behandlung von Krankheiten, die mit einer erhöhten Aktivität des Glucose-6-Phosphatase-Systems verbunden sind.

Die Erfindung betrifft daher auch die Anwendung von Verbindungen der Formel I bzw. der Salze zur Herstellung von Arzneimitteln zur Behandlung von Krankheiten, die mit einer erhöhten Glucoseproduktion der Leber verbunden sind.

Die Erfindung betrifft außerdem die Anwendung von Verbindungen der Formel I bzw. der Salze zur Herstellung von Arzneimitteln zur Behandlung des Typs II Diabetes (nicht insulinabhängiger oder Altersdiabetes).

Die Erfindung beinhaltet weiterhin die Anwendung von Verbindungen der Formel I bzw. der Salze zur Herstellung von Arzneimitteln zur Behandlung des Diabetes und anderer Erkrankungen, die durch einen erhöhten Glucose-Ausstoß aus der Leber oder eine erhöhte Aktivität des Glucose-6-Phosphatase-Systems gekennzeichnet sind.

Die Wirkung der erfindungsgemäßen Verbindungen auf das Glucose-6-Phosphatase-System wurde in einem Enzymtest in Lebermikrosomen untersucht.

Für die Präparation der die Glucose-6-Phosphatase enthaltenden Mikrosomen-Fraktion wurden frische Leber-Organe von männlichen Wistar-Ratten verwendet und wie in der Literatur beschrieben aufgearbeitet [Canfield, W. K. und Arion, W. J., J. Biol. Chem. 263, 7458 bis 7460, (1988)]. Diese Mikrosomen-Fraktion kann bei -70°C mindestens

2 Monate lang ohne signifikanten Aktivitätsverlust aufbewahrt werden.
Der Nachweis der Glucose-6-Phosphatase-Aktivität wurde wie in der Literatur angegeben (Arion, W. J. in Methods Enzymol. 174, Academic Press 1989, Seite 58 bis 67) durch Bestimmung des aus Glucose-6-Phosphat freigesetzten Phosphats durchgeführt. 0,1 ml Testansatz enthielten Glucose-6-Phosphat (1mMol/l), die Testsubstanz, 0,1 mg Mikrosomen-Fraktion und 100 mMol/l HEPES-Puffer (4-(2-Hydroxyethyl)piperazin-1-ethansulfonsäure), pH 7,0. Die Reaktion wurde durch Zugabe des Enzyms gestartet. Nach Ablauf von 20 Minuten bei Raumtemperatur wurde die Reaktion durch Zugabe von 0,2 ml Phosphat-Reagens gestoppt. Die Probe wurde 30 Minuten lang bei 37°C inkubiert, und die Absorbtion (A) der blauen Farbe anschließend bei 570 nm gemessen. Die inhibitorische Wirksamkeit der Testsubstanz ergab sich durch Vergleich mit einer Kontroll-Reaktion, die keine Testsubstanz enthielt nach der Formel

$$\text{Prozent Hemmung} = \frac{\text{A(Kontrolle) - A(Testsubstanz)}}{\text{A(Kontrolle)}} \times 100$$

Sofern erforderlich wurde die hemmende Wirkung der Testsubstanz als Funktion der eingesetzten Konzentration der Testsubstanz ermittelt, und daraus die Konzentration für die 50 %ige Hemmung der Enzymaktivität ($IC_{50}$) berechnet.

Für die nachfolgend aufgeführten Verbindungen wurde der $IC_{50}$-Wert ermittelt:

| Verbindung | $IC_{50}$ [μm]: |
|---|---|
| 4 | 0.02 |
| 9 | 0.3 |
| 19 | 0.8 |

Ein weiterer Gegenstand der Erfindung ist ein Arzneimittel, das ein oder mehrere erfindungsgemäße Verbindungen der Formel I und/oder ihre pharmakologisch verträglichen Salze enthält.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche, oder vorzugsweise in Kombination mit geeigneten pharmazeutischen Hilfsstoffen in Form von Tabletten, Dragees, Kapseln, Suppositorien, Emulsionen, Suspensionen, Granulaten, Pulvern, Lösungen oder Präparate mit protrahierter Wirkstoff-Freigabe, eingesetzt, wobei der Wirkstoffgehalt vorteilhafterweise 0,1 bis 95 % beträgt.

Welche Hilfstoffe für die gewünschte Arzneimittelformulierung geeignet sind, ist dem Fachmann aufgrund seines Fachwissens geläufig. Neben Lösungsmitteln, Gelbildnern, Suppositoriengrundlagen, Tabletten-Hilfsstoffen und anderen Wirkstoffträgern können beispielsweise Antioxidatien, Dispergiermittel, Emulgatoren, Entschäumer, Geschmackskorrigentien, Konservierungsmittel, Lösungsvermittler oder Farbstoffe verwendet werden.

Die Wirkstoffe können topisch, oral, parenteral oder intravenös appliziert werden, wobei die bevorzugte Applikationsart von der zu behandelnden Krankheit abhängig ist. Die orale Verabreichung ist bevorzugt.

Für eine orale Anwendungsform werden die aktiven Verbindungen mit den dafür geeigneten Zusatzstoffen wie Trägerstoffen, Stabilisatoren oder inerten Verdünndungsmittel vermischt und durch üblichen Methoden in geeigneten Dareichungsformen gebracht, wie Tabletten, Dragees, Steckkapseln, wässrige, alkoholische oder ölige Suspensionen oder wässrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen pflanzliche oder tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus verschiedenen Lösungsmitteln.

Als pharmazeutische Präparate für topische und lokale Verwendung eignen sich Augentropfen, welche die aktive Verbindung in wässriger oder öliger Lösung enthalten. Für die Anwendung an der Nase sind Aerosole und Sprays, sowie grobe Puder, die durch schnelles Inhalieren durch die Nasenlöcher verabreicht werden, und vor allem Nasentropfen, welche die aktive Verbindungen in wässriger oder öliger Lösung enthalten, geeignet.

Die Dosierung des zu verabreichenden Wirkstoffs der Formel I und die Häufigkeit der Verabreichung hängen von der Wirkstärke und Wirkdauer der verwendeten erfindungsgemäßen Verbindung ab; außerdem auch von der Art und Stärke der zu behandelnden Krankheit sowie von Geschlecht, Alter, Gewicht und individuellen Ansprechbarkeit des zu behandelnden Säugers. Im Durchschnitt liegt die empfohlene tägliche Dosis einer erfindungsgemäßen Verbindung bei einem etwa 75 Kg schweren Säuger - in erster Linier einem Menschen - im Bereich von etwa 1 bis 500 mg, vorzugweise von etwa 10 bis 250 mg, wobei die Verabreichung nach Bedarf in mehreren Dosen pro Tag erfolgen kann und gegebenenfalls auch niedriger oder höher sein kann.

Die Herstellung der erfindungsgemäßen Verbindungen der Formel I wird durch die Beispiele erläutert. Raumtemperatur bedeutet eine Temperatur von 20 bis 25°C.

Beispiel 1

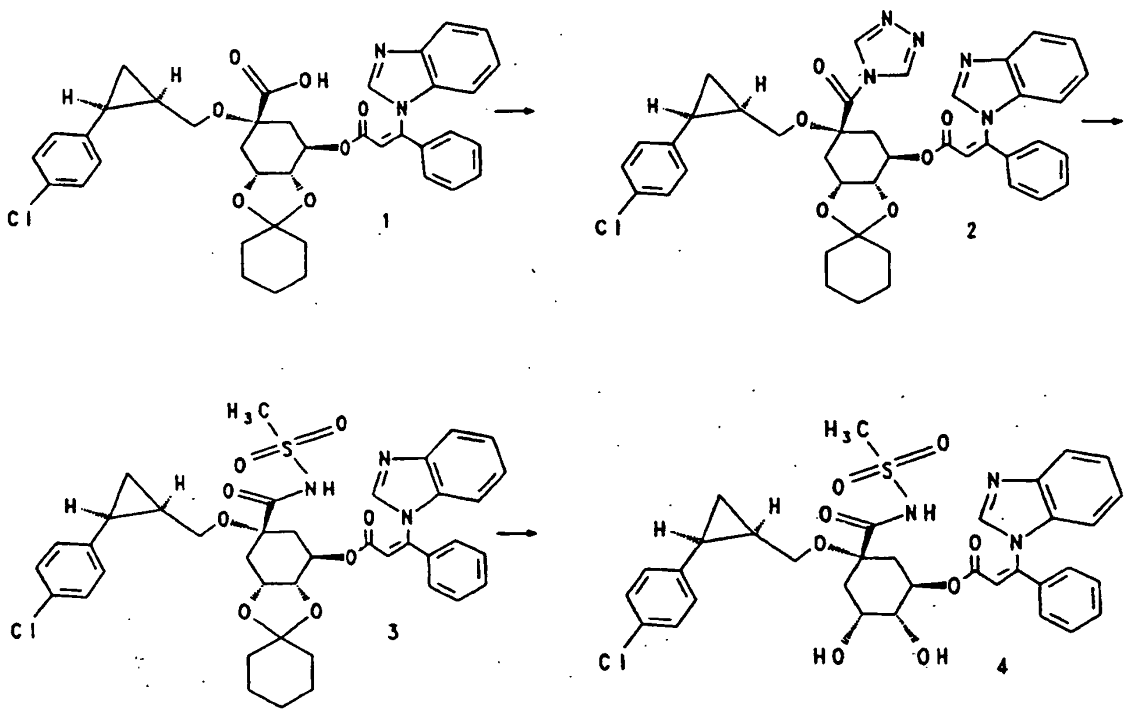

1 2 3 4

Darstellung von Verbindung 2 aus 1

3.7 g (0.054 Mol) der Carbonsäure 1 (Herstellung vergl. EP-Anmeldung Nr. 93 114 261.6, Reaktionsschema Verfahren A, Baustein 68B) wurden in 36 ml wasserfreiem Dimethylformamid gelöst und bei Raumtemperatur unter Argon mit 1.81 g (0.011 Mol) N,N'-Carbonyl-di-(1,2,4-triazol) versetzt und 1.5 Stunden auf 50 bis 60°C erwärmt. Nach dem Abkühlen wurde die 0.15 molare Lösung von 2 ohne weitere Aufarbeitung im Folgeschritt eingesetzt.

Darstellung von Verbindung 3 aus 2

0.057 g (0.006 Mol) Methansulfonamid wurden in 3 ml wasserfreiem Dimethylformamid gelöst und bei Raumtemperatur wurden 0.02 g (0.0066 Mol) Natriumhydrid (80 % in Öl) zugegeben. Die Suspension rührte man 45 Minuten bei 50 bis 60°C. Anschließend tropfte man bei dieser Temperatur 3.1 ml (0.00047 Mol) der 0.15 molaren Triazolidlösung 2 zu. Die Reaktionsmischung wurde bei 60°C 1 Stunde gerührt. Anschließend gab man die Reaktionsmischung auf gesättigte Ammoniumchlorid-Lösung, wobei das Produkt 3 als amorpher Feststoff ausfiel. Man saugte den Niederschlag ab ab, wusch mit dest. Wasser nach und trocknete den so erhaltenen Feststoff 3 Stunden bei $10^{-2}$ Torr und 40°C über Calciumchlorid . Man erhielt 0.248 g Verbindung 3.

Darstellung von Verbindung 4 aus 3

0.24 g (0.000316 Mol) Cyclohexylidenketal 3 wurden in 10 ml Dioxan vorgelegt und bei Raumtemperatur wurden unter kräftigem Rühren 1.6 ml (0.0032 Mol) 2 molarer Salzsäure zugegeben. Die klare Lösung rührte man 2 Stunden bei 50 bis 60°C. Anschließend kühlte man die Reaktionslösung auf 10 bis 20°C ab und titrierte die Reaktionslösung mit 1 molarer Natronlauge auf pH 3, verdünnte mit 20 ml dest. Wasser und engte die Reaktionsmischung im Vakuum soweit ein, daß kein Dioxan mehr abdestillierte. Beim Verrühren mit Wasser kristallisierte langsam ein Niederschlag, den man absaugte und mit Wasser wusch. Nach dem Trocknen bei 40°C im Hochvakuum erhielt man 0.18 g Verbindung 4 als farblosen Feststoff.

Auf diese Weise wurden folgende Verbindungen der Formel I synthetisiert:

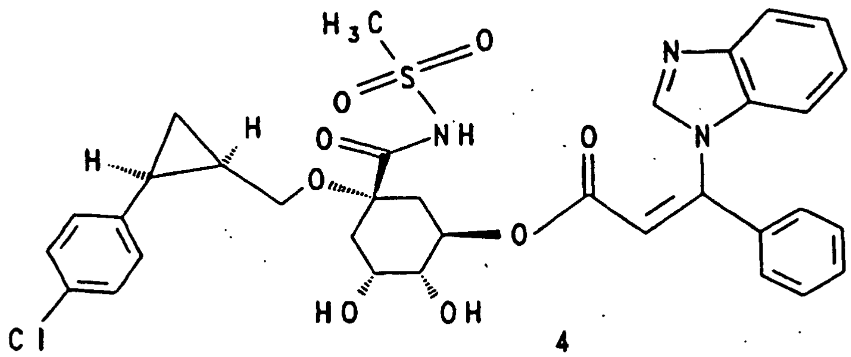

4

MS (FAB): m/z = 680 (M+H$^+$)

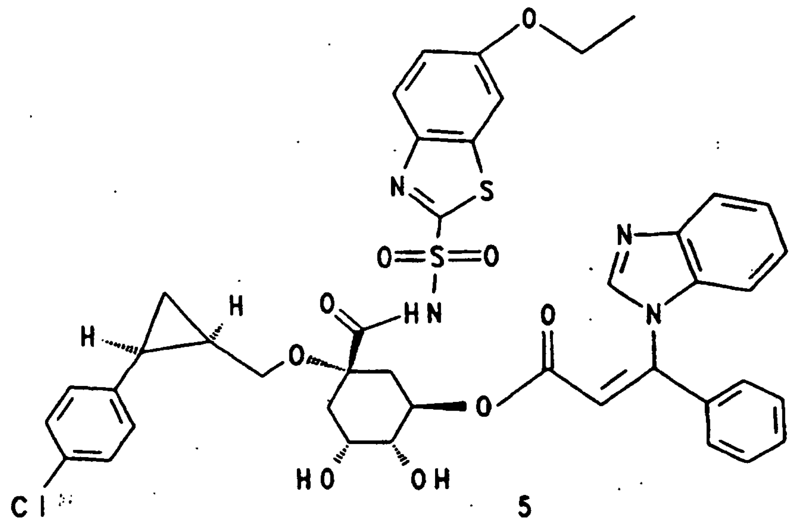

5

MS (FAB): m/z = 843 (M + H$^+$)

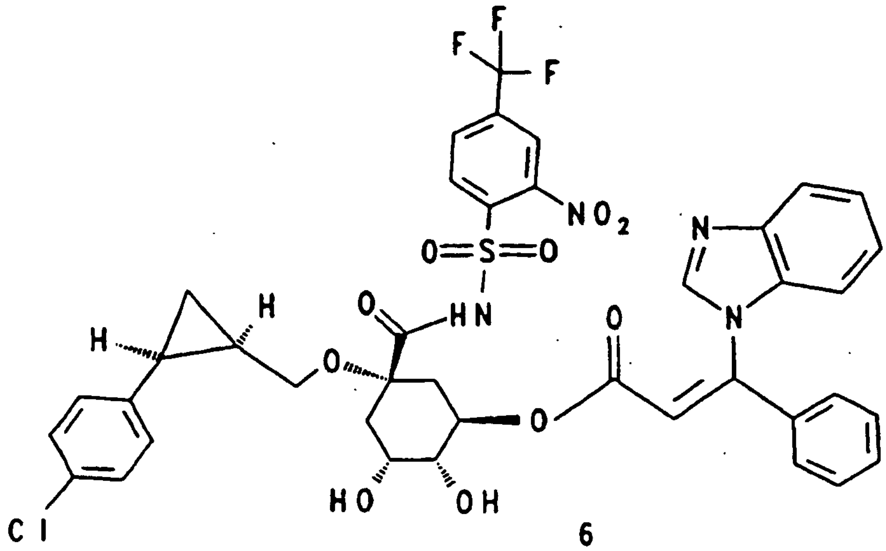

6

MS (FAB): m/z = 855 ($M+H^+$)

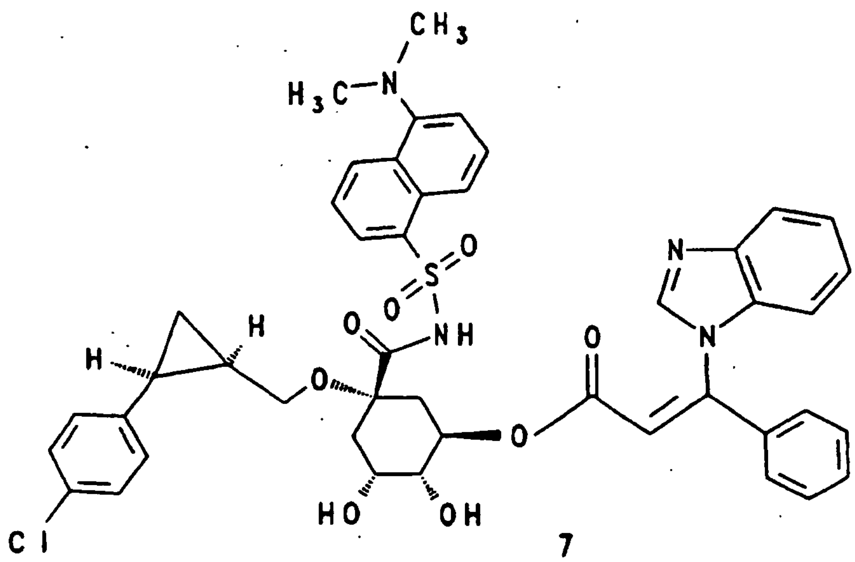

7

MS (FAB): m/z = 835 ($M+H^+$), 418 ($M + 2H^+$)

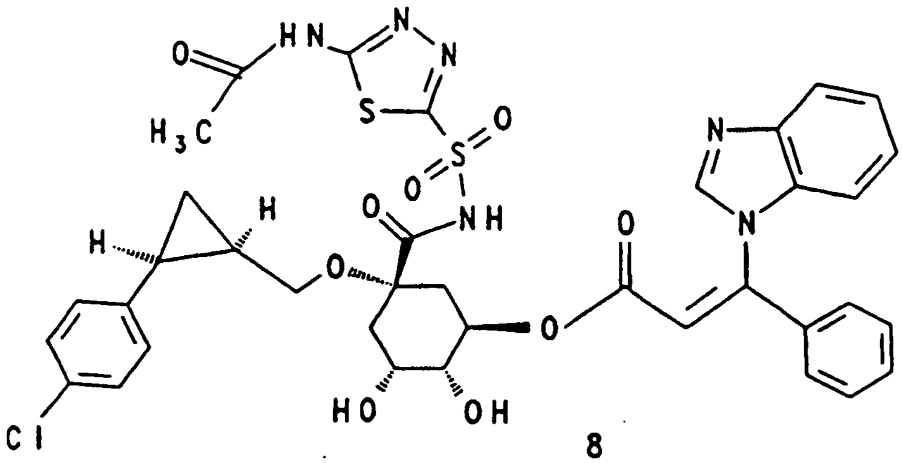

8

MS (FAB): m/z = 807 (M + $H^+$)

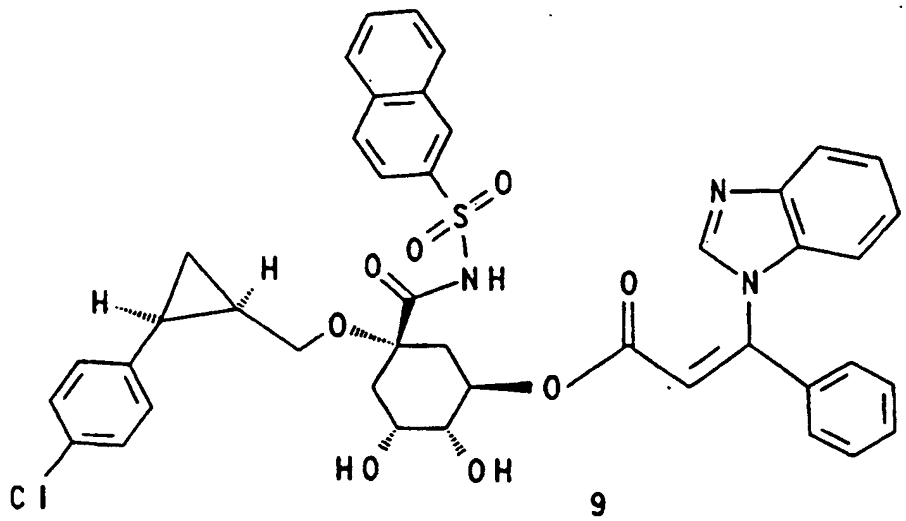

9

MS (FAB): m/z = 792 (M+$H^+$)

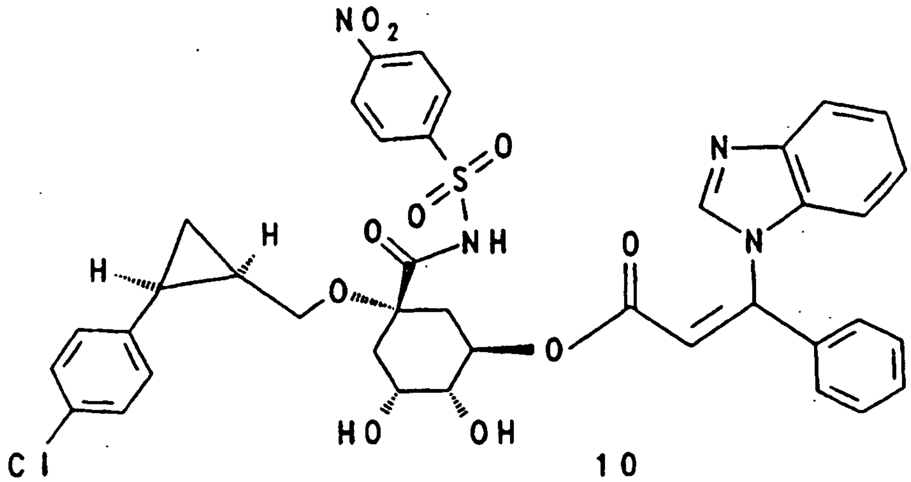

10

MS (FAB): m/z = 787 (M+H+)

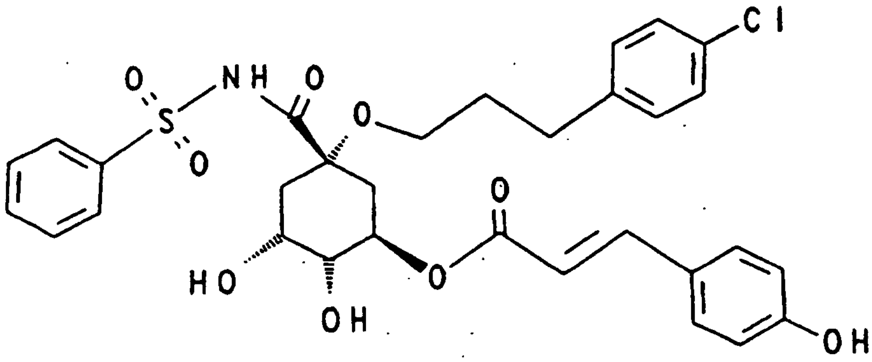

11

MS (FAB): m/z = 630 (M+H+)

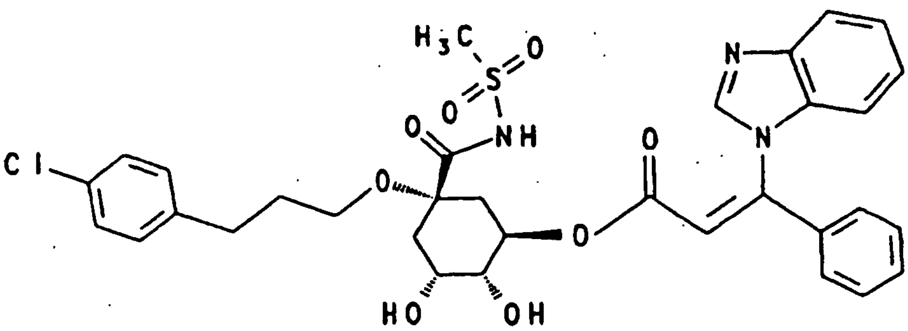

12

MS (FAB): m/z = 668 (M+H+)

Beispiel 2

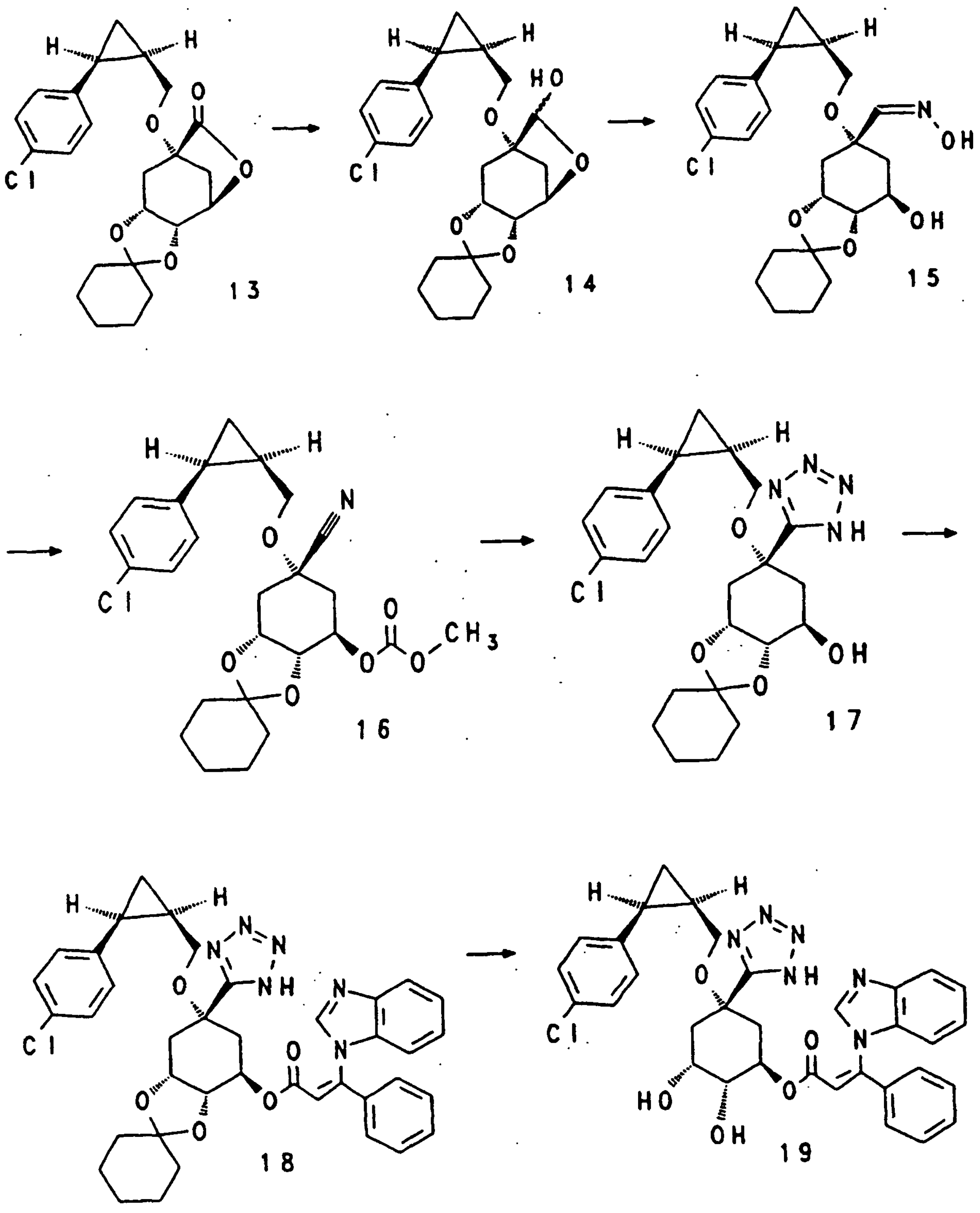

13 14 15 16 17 18 19

Darstellung von Verbindung 14 aus 13:

5.0 g (0.012 Mol) Lacton 13 (Herstellung vergl. EP-Anmeldung Nr. 93 114 261.6, Reaktionsschema Verfahren A, Baustein 68B) wurden in 80 ml wasserfreiem Toluol gelöst und bei -78°C wurden unter Argon-Atmosphäre 10 ml (0.012 Mol) einer 1.2 molaren Diisobutylaluminiumhydrid-Lösung in Hexan zugetropft. Nach 1 Stunde bei -50°C wurde mit gesättigter Ammoniumchlorid-Lösung hydrolisiert. Man extrahierte mit Essigester, wusch die vereinigten organischen Phasen mit gesättigter Natriumchlorid-Lösung und trocknete mit Magnesiumsulfat. Die organische Phase wurde

im Vakuum eingeengt und das so erhaltene Lactol 14 ohne weitere Reinigung im Folgeschritt eingesetzt.

Darstellung von Verbindung 15 aus 14:

4.6 g (0.011 Mol) Lactol 14 und 0.761 g (0.011 Mol) Hydroxylamin Hydrochlorid wurden in 50 ml Methanol gelöst. Es wurden 750 mg (0.014 Mol) Kaliumhydroxid zugegeben. Diese Lösung wurde 1 Stunde bei Raumtemperatur gerührt. Anschließend versetzte man die Lösung mit 300 ml Methyl-tert-butylether, wusch mit Wasser und gesättigter Natriumchlorid-Lösung und engte die organische Phase nach Trocknen mit Magnesiumsulfat im Vakuum ein. Der Rückstand wurde durch Chromatographie an Kieselgel (Laufmittel: Essigester/n-Heptan 1:2) gereinigt. Man erhielt 3.6 g Oxim 15 als farbloses Öl.

Darstellung von Verbindung 16 aus 15:

20.0 g (0.046 Mol) Oxim 15 wurden in 200 ml wasserfreiem Dichlormethan vorgelegt und es wurden 23.0 g (0.14 Mol) N,N'-Carbonyldimidazol zugegeben. Es folgt eine starke Gasentwicklung. Nach 14 Stunden bei Raumtemperatur wurde zur Reaktionslösung 100 ml Methanol gegeben und weitere 4 Stunden unter Rückfluß erwärmt. Zur Aufarbeitung wurde die Lösung bis zur Trockne einrotiert und in Methyl-tert-butylether aufgenommen. Die organische Phase wurde mit einer Mischung Wasser/0.1 M Kaliumhydrogensulfat-Lösung gewaschen, mit Magnesiumsulfat getrocknet und im Vakuum eingeengt. Den Rückstand reinigte man durch Chromatographie an Kieselgel (Kieselgelkorngröße 35 bis 70 µm, Laufmittelsystem: Essigester/n-Heptan 1:5, gegen Ende wurde der n-Heptan-Anteil erniedrigt: 1: 3). Man erhielt 12.9 g Nitril 16 als farbloses Öl.

Darstellung von Verbindung 17 aus 16:

12.9 g (0.0286 Mol) Nitril 16 wurden in 250 ml wasserfreiem Toluol gelöst und auf 110°C erhitzt. Über drei Tage wurden im Abstand von 24 Stunden jeweils 5.89 g (0.0286 Mol) Trimethylzinnazid zugegeben. Anschließend engte man die Reaktionslösung im Vakuum ein und versetzte den Rückstand unter kräftigem Rühren mit 50 ml 10 molare Natronlauge und 20 ml Tetrahydrofuran. Das entstandene Natriumsalz von 17 wurde abgesaugt, danach in dest. Wasser suspendiert und mit 2 molarer Essigsäure angesäuert. Man extrahierte mit Essigester, trocknete die vereinigten organischen Phasen mit Magnesiumsulfat und engte im Vakuum ein. Man erhielt 7.7 g Tetrazol 17.

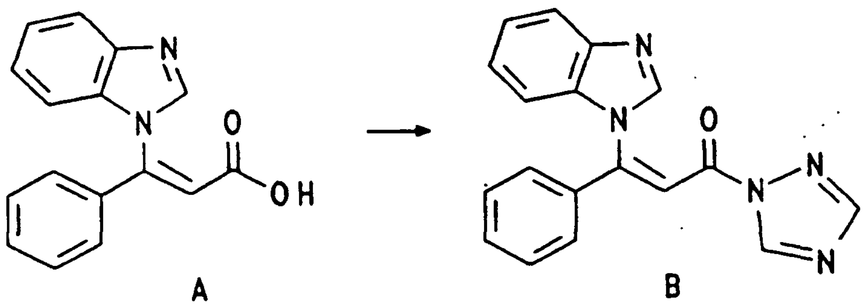

A B

Darstellung von Ausgangsverbindung B aus A:

274 mg (0.001 Mol) Carbonsäure A (Herstellung vergl. EP-Anmeldung Nr. 93 114 261.6, Verfahren I) wurden unter Argon-Atmosphäre bei Raumtemperatur in 20 ml wasserfreiem Dimethylformamid gelöst und es wurden 180.4 mg (0.0011 Mol) N,N'-Carbonyl-di-(1,2,4-triazol) zugegeben. Die Reaktionslösung rührte man 1 Stunde bei 60°C. Die erhaltene Lösung von Verbindung B wurde ohne weitere Aufarbeitung im Folgeschritt eingesetzt.

Darstellung von Verbindung 18 aus 17:

3.0 g (0.00652 Mol) Verbindung 17 wurden in 30 ml wasserfreiem Dimethylformamid unter Argon-Atmosphäre gelöst und bei Raumtemperatur wurden 0.70 g (0.023 Mol) Natriumhydrid (80 % Dispersion in Öl) zugegeben. Nach 1 Stunde tropfte man 157 ml (0.0078 Mol) einer 0.5 molaren Lösung von B in Dimethylformamid zu und rührte wiederum 1 Stunde bei Raumtemperatur. Anschließend wurde die Reaktionslösung auf gesättigte Ammoniumchlorid-Lösung gegeben und mit Essigester extrahiert. Die vereinigten organischen Phasen wurden mit gesättigter Natriumchlorid-Lösung gewaschen, über Magnesiumsulfat getrocknet und im Vakuum eingeengt. Das Rohprodukt wurde chromatographisch gereinigt. (Kieselgelkorngröße: 35 bis 70 µm, Laufmittelsystem: Essigester/n-Heptan/Methanol/Eisessig 30: 10:2:1). Man erhielt den Ester 18 als amorphen Feststoff.

Darstellung von Verbindung 19 aus 18:

3.8 g (0.0053 Mol) Cyclohexylidenverbindung 18 wurden in 150 ml Dioxan aufgenommen und unter Rühren mit 10 ml (0.02 Mol) 2 molarer Salzsäure versetzt. Diese Lösung erhitzte man 2 Stunden auf 60°C. Anschließend stellte man den pH-Wert der Reaktionslösung mit 18 ml 1N molarer Natronlauge auf 3 ein und rotierte das Lösungsmittel ab. Der Rückstand wurde in Essigester aufgenommen und der ausgefallene Niederschlag abfiltriert. Das Filtrat engte man im Vakuum ein und reinigte den Rückstand durch Chromatographie an Kieselgel (Kieselgelkorngröße 35 bis 70 µm, Laufmittelsystem: Essigester/Methanol/Wasser/Eisessig 4:1:1:0.5). Man erhielt 2.5 g Verbindung 19 als farblosen amorphen Feststoff.

Auf diese Weise wurden folgende Verbindungen der Formel I synthetisiert:

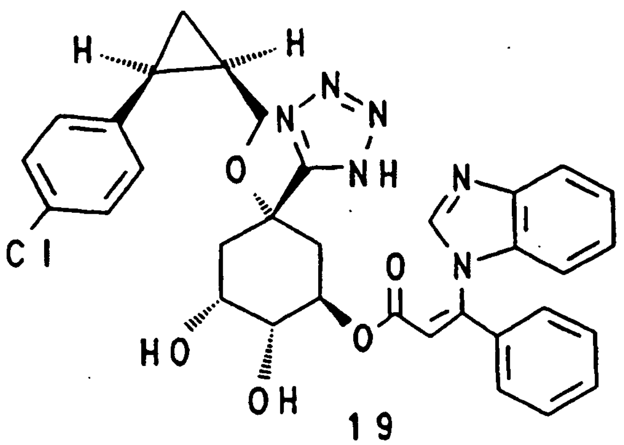

19

MS (FAB): m/z = 627 (M+H$^+$)

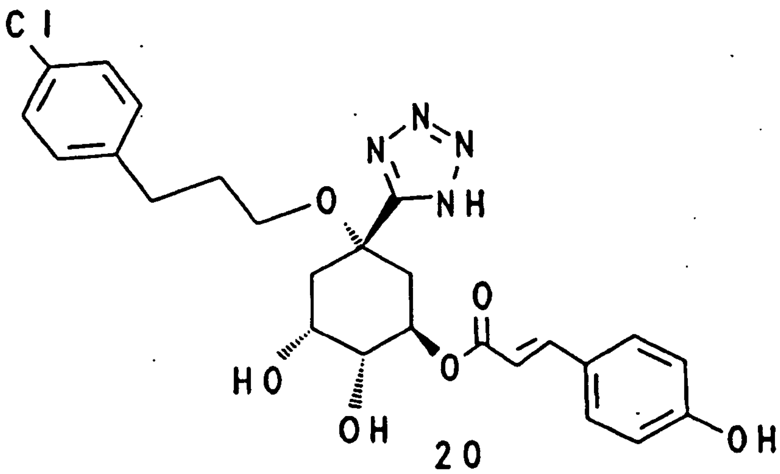

20

MS (FAB): m/z = 515 (M+H$^+$)

Beispiel 3

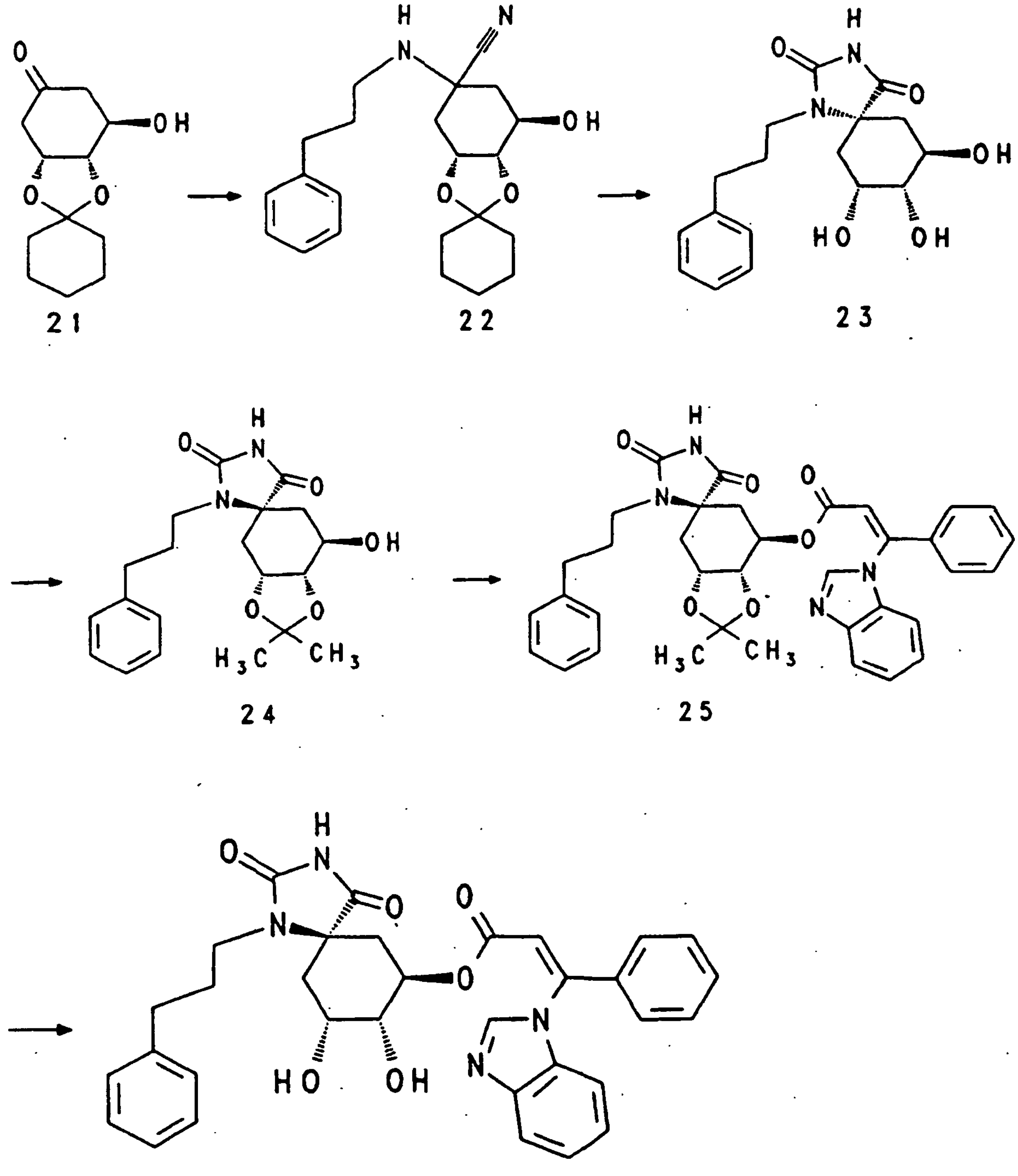

21 22 23 24 25

26

Darstellung von Verbindung 22 aus 21:

2.26 g (0.01 Mol) des literaturbekannten Ketons 21 (vergl. J. C. Barrier et al., Helv. Chim. Acta 66, 296 (1983)) und 4.29 g (0.025 Mol) 3-Phenylpropylaminhydrochlorid wurden unter Argon-Atmosphäre in 5 ml Methanol und 3 ml dest. Wasser vorgelegt. Die Mischung wurde auf 0°C gekühlt und eine Lösung aus 1.63 g (0.025 Mol) Kaliumcyanid in 4 ml dest. Wasser wurde zugetropft. Die Reaktionsmischung wurde 4 Stunden bei 0°C und 1 Stunde bei Raumtemperatur gerührt und anschließend unter Rühren auf Eis/Wasser gegeben und mit Essigester dreimal extrahiert. Die vereinigten organischen Phasen wusch man dreimal mit dest. Wasser und einmal mit gesättigter Natriumchlorid-Lösung, trocknete mit Magnesiumsulfat und engte im Vakuum ein. Man erhielt 5.0 g Rohprodukt 22, das ohne weitere Reinigung im Folgeschritt eingesetzt wurde.

Darstellung von Verbindung 23 aus 22:

3.7 g (0.01 Mol) Cyanoverbindung 22 wurden in 8 ml Eisessig gelöst und unter Rühren bei Raumtemperatur mit einer Lösung von 1.62 g (0.02 Mol) Kaliumcyanat in 4 ml dest. Wasser versetzt. Die Reaktionslösung wurde 75 Minuten bei Raumtemperatur gerührt und anschließend auf eine Mischung aus Eis und Wasser gegeben, zweimal mit Essigester extrahiert und die vereinigten organischen Phasen wurden einmal mit dest. Wasser und einmal mit gesättigter Natriumchlorid-Lösung gewaschen. Nach dem Trocknen der organischen Phase mit Magnesiumsulfat engte man im Vakuum ein, löste den so erhaltenen öligen Rückstand in 4 ml Dioxan und versetzte diese Lösung unter Rühren mit 10 ml 2 molarer Salzsäure. Nach einer Stunde Rühren bei 55°C wurde die Reaktionsmischung auf ein Eis-Wasser-Gemisch gegossen und dreimal mit Essigester extrahiert. Die organischen Phasen wurden dreimal mit Wasser und einmal mit gesättigter Natriumchloridlösung gewaschen, über Magnesiumsulfat getrocknet und im Vakuum eingeengt. Den öligen Rückstand reinigte man durch Chromatographie an Kieselgel (Kieselgelkorngröße: 35 bis 70 µm, Laufmittelsystem: Essigester/n-Heptan/Methanol/Eisessig 20:10:2:1) und man erhielt 0.36 g Produkt 23.

Darstelllung von Verbindung 24 aus 23:

0.36 g (0.00106 Mol) Verbindung 23 wurden in 1.09 ml (0.0106 Mol) Dimethoxypropan und 20 ml wasserfreiem Dichlormethan gelöst. Es wurden 26 mg (10 Mol-%) Pyridin-para-toluolsulfonat zugegeben. Die Mischung wurde 45 Minuten auf 40°C erwärmt. Anschließend gab man die Reaktionslösung auf gesättigte Natriumhydrogensulfatlösung, extrahierte mit Essigester und trocknete die vereinigten organischen Phasen mit Magnesiumsulfat. Den Rückstand reinigte man durch Chromatographie an Kieselgel (Kieselgelkorngröße: 35 bis 70 µm, Laufmittelsystem: Essigester/n-Heptan 2:1) und erhielt 0.24 g Verbindung 24 als farblosen Feststoff.

Darstellung von Verbindung 25 aus 24:

230 mg (0.0006 Mol) Hydroxyverbindung 24 wurden in 10 ml wasserfreiem Dimethylformamid gelöst und bei Raumtemperatur unter Argon-Atmosphäre 55 mg (0.00184 Mol) Natriumhydrid (80 % Dispersion in Öl) zugegeben. Nach 30 Minuten bei Raumtemperatur tropfte man 22 ml einer 0.5 molaren Lösung von B in Dimethylformamid zu. Nach weiteren 30 Minuten bei dieser Temperatur erhielt man eine klaren Lösung, die mit gesättigter Ammoniumchlorid-Lösung versetzt wurde, wobei das Produkt 25 als amorpher Feststoff ausfiel. Dieser wurde abgesaugt und im Vakuum getrocknet. Man erhielt 310 mg Verbindung 25.

Darstellung von Verbindung 26 aus 25:

290 mg (0.00047 Mol) Verbindung 25 wurden in 30 ml Dioxan gelöst und bei Raumtemperatur unter kräftigem Rühren mit 4 ml (0.008 Mol) 2 molarer Salzsäure versetzt. Nach zwei Stunden Rühren bei 50°C kühlte man die Reaktionslösung auf 10 bis 20°C ab und titrierte mit 1 molarer Natronlauge auf pH 3. Man engte die Lösung im Vakuum ein und nahm den öligen Rückstand in Isopropanol auf, filtrierte den Salzniederschlag ab und engte das Filtrat wiederum im Vakuum ein. Den Rückstand verührte man mit Methyl-tert-butylether und saugte den amorphen Niederschlag ab. Man erhielt nach dem Trocknen im Vakuum 140 mg Verbindung 26 (Endprodukt der Formel I).

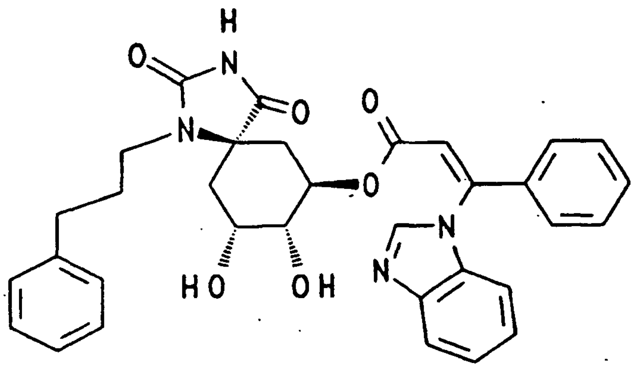

2 6

MS (FAB): m/z = 581 (M+H+)

Beispiel 4

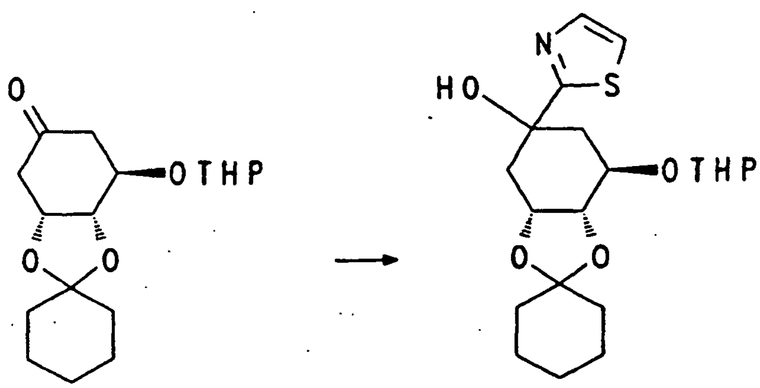

2 7 2 8

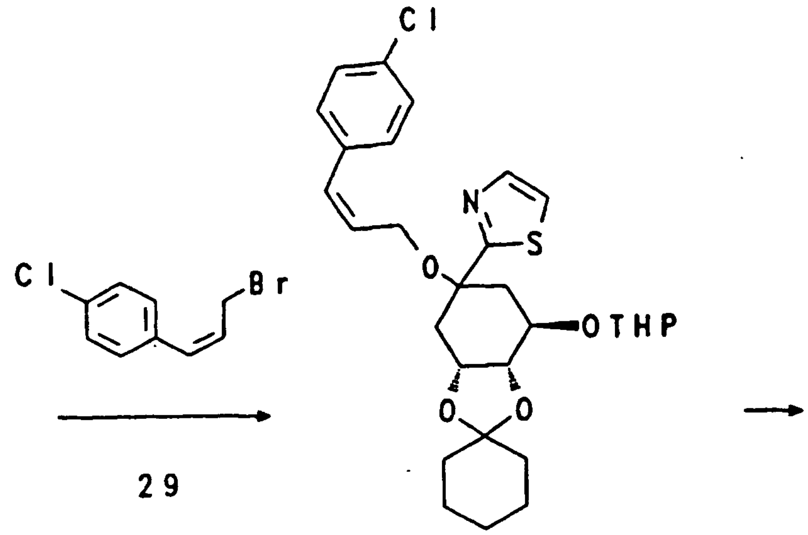

29

30

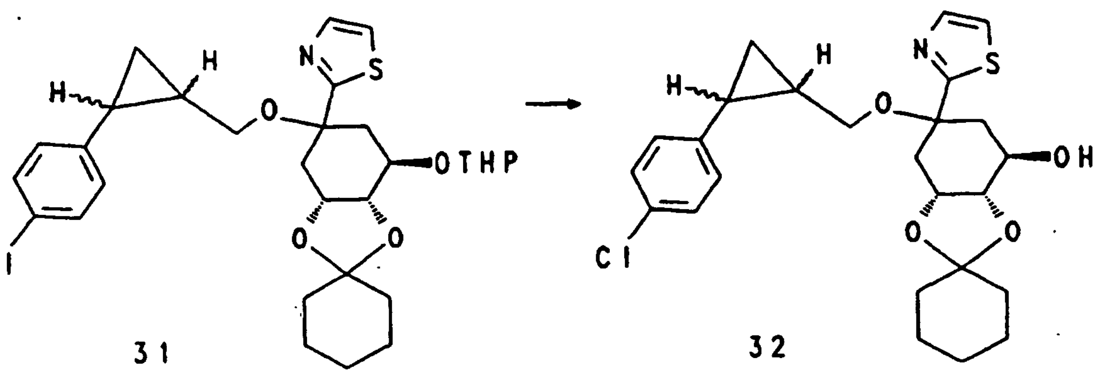

31

32

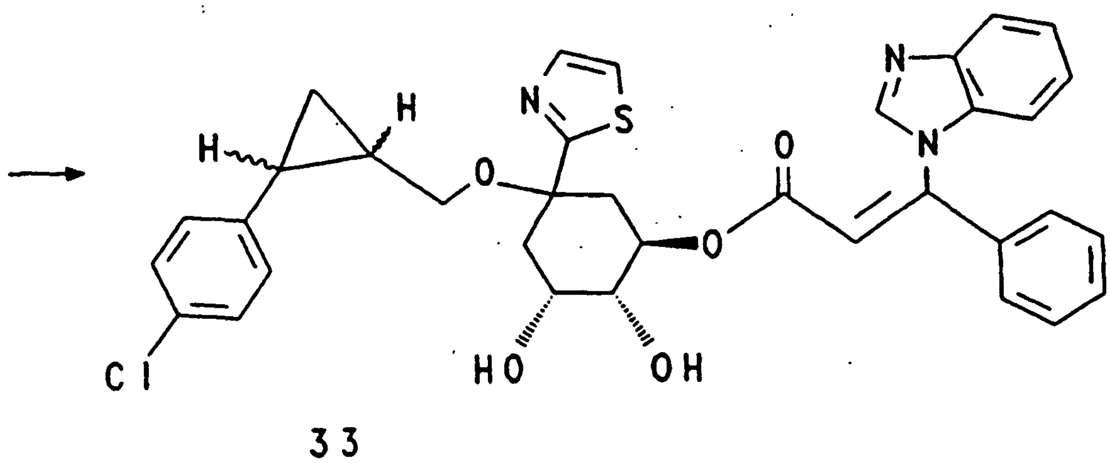

33

Darstellung von Verbindung 28 aus 27:

26.4 g (14,5 ml, 0.161 Mol) 2-Bromthiazol wurden in 500 ml wasserfreiem Diethylether unter Argon-Atmosphäre gelöst und bei -78°C wurden 107.5 ml n-Butyllithium in Hexan (1.5 molare Lösung) zugetropft. Man rührte 30 Minuten bei -78°C und tropfte anschließend eine Lösung von 25.0 g (0.081 Mol) Keton 27 (vergl. EP-Anmeldung Nr. 92 114 260.8, Formelschema 4, Verbindung 23B) in 50 ml wasserfreiem Tetrahydrofuran zu. Man ließ die Reaktionslösung innerhalb von 30 Minuten auf -30°C aufwärmen. Danach wurde die Reaktionslösung auf Ammoniumchlorid-Lösung gegeben, mit Essigester extrahiert und die vereinigten organischen Phasen mit gesättigter Natriumchlorid-Lösung gewaschen und mit Natriumsulfat getrocknet. Die organische Phase wurde im Vakuum eingeengt und der Rückstand durch Chromatographie an Kieselgel (Laufmittel: Essigester/Heptan 1:2, Korngröße: 35 bis 70 µm) gereinigt. Man erhielt 23.3 g (77 %) Verbindung 28 als zähes Öl.

Darstellung von Verbindung 30 aus 28:

15.0 g (0.038 Mol) Alkohol 28 wurden in 250 ml wasserfreiem Dimethylformamid gelöst und bei 0 bis 10°C wurden 1.5 g (0.05 Mol) Natriumhydrid zugegeben. Man rührte 1.5 Stunden bei 10°C, kühlte anschließend auf 0°C ab und tropfte 13.2 g (0.057 Mol) cis-3-(4-Chlorphenyl)-propenylbromid (29), gelöst in 30 ml wasserfreiem Dimethylformamid, zu. Man ließ die Reaktionslösung auf Raumtemperatur aufwärmen und rührte 2 Stunden bei dieser Temperatur. Anschließend wurde die Reaktionslösung auf gesättigte Ammoniumchlorid-Lösung gegeben, mit Essigester extrahiert und die vereinigten organischen Phasen wurden mit gesättigter Natriumchlorid-Lösung gewaschen. Nach dem Trocknen mit Natrumsulfat wurde die organische Phase im Vakuum eingeengt und durch Chromatographie an Kieselgel (Laufmittel: Essigester/n-Heptan 1:2, Korngröße 35 bis 70 µm) gereinigt. Man erhielt 19.0 g Thiazol 30 als zähes Öl.

Darstellung von Verbindung 31 aus 30:

56.6 ml einer 1.1 molaren Lösung von Diethylzink in Toluol wurden bei 0°C unter Argon-Atmosphäre in 250 ml wasserfreiem Dichlorethan getropft und anschließend wurden 9.0 ml (0.125 Mol) Chloriodmethan bei 0°C zugegeben. Man rührte die Reaktionslösung 30 Minuten bei gleicher Temperatur und tropfte danach 17.0 g (0.031 Mol) Olefin 30, gelöst in 30 ml wasserfreiem Dichlorethan, zu. Man ließ langsam auf Raumtemperatur aufwärmen. Nach 2 Stunden wurde die Reaktionslösung auf gesättigte Ammoniumchlorid-Lösung gegeben, mit Essigester extrahiert und die vereinigten organischen Phasen wurden mit gesättigter Natriumchlorid-Lösung gewaschen. Nach dem Trocknen der organischen Phase wurde mit Natriumsulfat im Vakuum eingeengt und der Rückstand mit Methyl-tert.-butylether ausgerührt. Man filtriert den Niederschlag ab (als Nebenreaktion trat Methylierung des Stickstoffs im Thiazolring auf) und engte das Filtrat erneut ein. Man erhielt 4.2 g (24 %) Verbindung 31 als zähes Öl.

Darstellung von Verbindung 32 aus 31:

4.2 g (0.008 Mol) 31 wurde in 100 ml Methanol und 150 ml Dichlormethan gelöst und bei Raumtemperatur wurden 0.7 g (0.003 Mol) Pyridiniump-toluolsulfonat zugegeben. Die klare Lösung ließ man 14 Stunden bei Raumtemperatur stehen, versetzte mit 20 ml 1 N NatriumhydrogencarbonatLösung und engte diese Mischung soweit ein, bis nur noch die wässrige Phase zurückblieb. Diese wurde mit Essigester extrahiert, und die vereinigten organischen Phasen wurden mit gesättigter Natriumchlorid-Lösung gewaschen, mit Natriumsulfat getrocknet und im Vakuum eingeengt. Der Rückstand wurde durch Chromatographie an Kieselgel (Laufmittel: Essigester/n-Heptan 1:1, Korngröße 35 bis 70 µm) gereinigt. Man erhielt 1.82 g (51 %) Verbindung 32 als farbloses Öl.

Darstellung von Verbindung 33 aus 32:

Analog der in Beispiel 2 beschriebenen Herstellung von Verbindung 19 aus Verbindung 17 erhielt man aus 32 über 2 Stufen Verbindung 33 der Formel I als amorphen Feststoff.
MS (FAB): m/z = 542 ($M+H^+$)

**Patentansprüche**

1. Cyclohexanol-Ester der Formel I

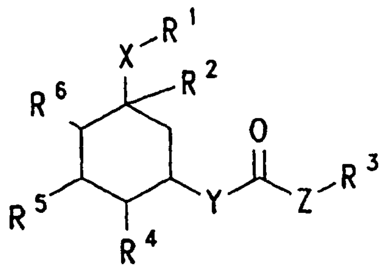

I

worin die Reste die folgende Bedeutung haben:

$R^1$: $CONHCOR^{15}$, $CSNHR^{15}$, $CONHSO_2R^{14}$, $CSNHSO_2R^{14}$ oder $CH_2NHSO_2R^{14}$ oder einen Rest der folgenden Formeln:

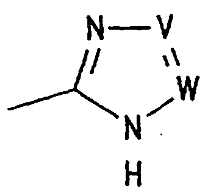 , 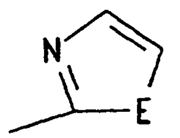 , 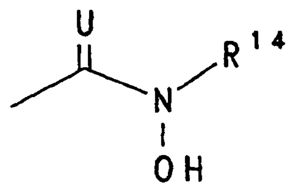 ,

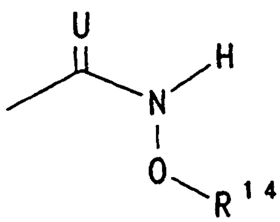 , 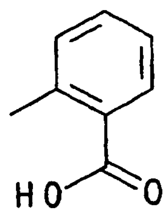 oder 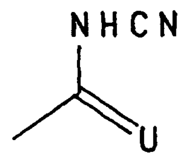

worin E = O, S oder NH, $R^{14}$ = H, U = O oder S, V = N oder CH, W = N oder CH bedeuten und aromatische Ringe einfach oder mehrfach mit F, Cl, Br, I, OH, O-$C_1$-$C_4$-Alkyl, $C_1$-$C_4$-Alkyl, $CF_3$, $NO_2$, CN, substituiert sein können,
oder $R^1$ bildet gemeinsam mit $R^2$ den Ring

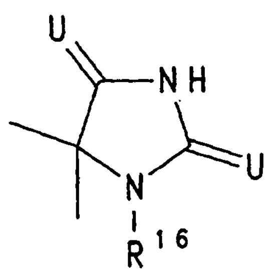

$R^2$: O-$C_1$-$C_{10}$-Alkyl$(R^{11})_n$, (n = 0, 1, 2), wobei der Alkylteil unverzweigt, verzweigt oder cyclisch ist, und $R^{11}$ mit $R^{12}$ substituiert sein kann und bei n = 2 die beiden Reste $R^{11}$ gleich oder verschieden sind, O-$C_3$-$C_{10}$-Alkenyl$(R^{11})_n$ (n = 0, 1, 2), wobei der Alkenylteil unverzweigt, verzweigt oder cyclisch ist, und ein- oder mehrfach ungesättigt ist, und $R^{11}$ mit $R^{12}$ substituiert sein kann und bei n = 2 die beiden Reste $R^{11}$ gleich oder

verschieden sind,
$O-C_3-C_{10}$-Alkinyl$(R^{11})_n$, (n = 0, 1, 2), wobei der Alkinylteil unverzweigt, verzweigt oder cyclisch ist und ein- oder mehrfach ungesättigt ist und $R^{11}$ mit $R^{12}$ substituiert sein kann,

$R^3$, R" und $R^{13}$: Alkyl mit 1 bis 10 C-Atomen, Cycloalkyl mit 3 bis 8 Ring-C-Atomen, Phenyl, Naphthyl, Phenanthryl, Pyridyl, Thienyl, Furyl, Pyrimidyl, Indolyl, Imidazolyl, Cumarinyl, Phthaliminyl, Chinolyl, Piperazinyl, Tetrazolyl, Triazolyl, Oxazolyl oder deren thieno-, pyridino-, pyrimidino- oder benzoanellierte Derivate, wobei der Aromat bzw. Heteroaromat ein- oder mehrfach mit F, Cl, Br, I, OH, $-NO_2$, CN, $C_1-C_4$-Alkoxy, $C_1-C_4$-Alkyl, $NR^8R^9$, Phenyl, Benzyl, Thienyl, Furyl, Imidazolyl, Pyridyl, O-Phenyl oder O-Benzyl gleich oder verschieden substituiert sein kann, und $R^3$, $R^{11}$ und $R^{13}$ gleich oder verschieden sind;

$R^4$, $R^5$ und $R^6$: H, OH, eine durch übliche Alkoholschutzgruppen geschützte OH-Gruppe, wobei $R^4$, $R^5$ und $R^6$ gleich oder verschieden sind;

$R^8$ und $R^9$: H, $C_1-C_4$-Alkyl, $C_1-C_4$-Alkanoyl, Phenyl, das gegebenenfalls substituiert ist mit F, Cl, Br, I, OH, $O-C_1-C_4$-Alkyl, $CF_3$, $-NO_2$ oder CN, wobei $R^8$ und $R^9$ gleich oder verschieden sind, oder $R^8$ und $R^9$ bilden zusammen mit dem Stickstoffatom einen 4 bis 10gliedrigen, gesättigten heterocyclischen Ring, bei dem gegebenenfalls eine $CH_2$-Gruppe durch O, S oder $NR^{10}$ ersetzt sein kann, $R^{10}$: H, $C_1-C_4$-Alkyl, Phenyl oder Benzyl

$R^{12}$: Phenyl, Naphthyl, Phenanthryl, Pyridyl, Thienyl, Furyl, Thiazolyl, Pyrimidyl, Indolyl, Imidazolyl, Coumarinyl, Phthaliminyl, Chinolyl, Piperazinyl, Tetrazolyl, Triazolyl, Oxazolyl oder deren thieno- oder benzoanellierte Derivate, wobei der Aromat bzw. Heteroaromat ein- oder mehrfach mit F, Cl, Br, I, OH, $CF_3$, $-NO_2$, CN, $C_1-C_4$-Alkoxy, $C_1-C_4$-Alkyl, $NR^8R^9$, Phenyl, Benzyl, Thienyl, Furyl, Imidazolyl, Pyridyl, O-Phenyl oder O-Benzyl gleich oder verschieden substituiert sein kann;

$R^{14}$: Wasserstoff, $C_1-C_{10}$-Alkyl, Phenyl, Naphthyl, Phenanthryl, Pyridyl, Thienyl, Furyl, Thiazolyl, Pyrimidyl, Indolyl, Imidazolyl, Coumarinyl, Phthaliminyl, Chinolyl, Piperazinyl, Tetrazolyl, Triazolyl, Oxazolyl oder deren thieno- oder benzoanellierte Derivate, wobei der Aromat bzw. Heteroaromat ein- oder mehrfach mit F, Cl, Br, I, OH, $CF_3$, $-NO_2$, CN, $C_1-C_4$-Alkoxy, $C_1-C_4$-Alkyl, $NR^8R^9$, Phenyl, Benzyl, Thienyl, Furyl, Imidazolyl, Pyridyl, O-Phenyl oder O-Benzyl gleich oder verschieden substituiert sein kann oder $R^{14}$ ist ein Rest der Formel

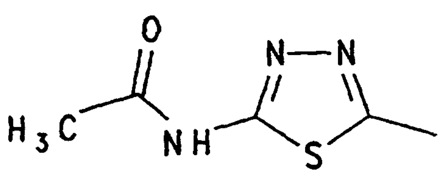

$R^{15}$: $C_3-C_{10}$-Alkenoyl, $C_3-C_{10}$-Alkenoyl$(R^{12})$, $C_1-C_{10}$-Alkanoyl$(R^{12})$, Phenyl, Naphthyl, Phenanthryl, Pyridyl, Thienyl, Furyl, Thiazolyl, Pyrimidyl, Indolyl, Imidazolyl, Coumarinyl, Phthaliminyl, Chinolyl, Piperazinyl, Tetrazolyl, Triazolyl, Oxazolyl oder deren thieno- oder benzoanellierte Derivate, wobei der Aromat bzw. Heteroaromat ein- oder mehrfach mit F, Cl, Br, I, OH, $CF_3$, $-NO_2$, CN, $C_1-C_4$-Alkoxy, $C_1-C_4$-Alkyl, $NR^8R^9$, Phenyl, Benzyl, Thienyl, Furyl, Imidazolyl, Pyridyl, O-Phenyl oder O-Benzyl gleich oder verschieden substituiert sein kann;

$R^{16}$: $C_1-C_{10}$-Alkyl$(R^{11})$n, $C_3-C_{10}$-Alkenyl$(R^{11})$n oder $C_3-C_{10}$-Alkinyl$(R^{11})$n mit n jeweils null oder eins;

X: $(CH_2)_m$, (m = 0, 1, 2, 3, 4) CH = CH, C≡C, $CH_2OCH_2$ oder $CH_2SCH_2$;

Y: $(CH_2)_m$, (m = 0, 1, 2, 3, 4) O, S oder $NR^8$;

Z: $(CH_2)_m$, (m = 0, 1, 2, 3, 4) S, O, $S-C_1-C_{10}$-Alkyl, CH = CH, CH = CF, CH = CCl, CH=CBr, $CH_2$-C(O), $CH_2$-CHF, $CH_2$-CHCl, $CH_2$-CHBr, $CH_2$-CHI, $C_3-C_{10}$-Cycloalkylen, $C_3-C_{10}$-Cycloalkenylen, $COOR^7$, C≡C, CH = $C(C_1-C_4$-Alkyl) CH = C(CN), CH = $C(R^{13})$ oder $NR^8$.

**2.** Cyclohexanol-Ester und deren physiologisch verträgliche Salze gemäß Anspruch 1, **dadurch gekennzeichnet, daß** die Reste in Formel I folgende Bedeutungen haben:

$R^1$: $CONHCOR^{15}$, $CSNHR^{15}$, $CONHSO_2R^{14}$, $CSNHSO_2R^{14}$ oder einen Rest der Formeln:

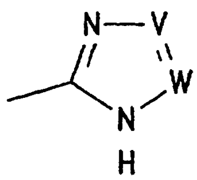 , 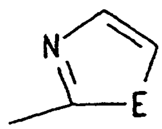 , 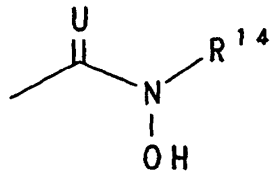 oder 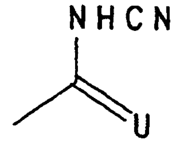

worin E = O, S oder NH, $R^{14}$ = H, U = O oder S, V = N oder CH, W = N oder CH bedeuten und aromatische Ringe einfach oder mehrfach mit F, Cl, Br, I, OH, O-$C_1$-$C_4$-Alkyl, $C_1$-$C_4$-Alkyl, $CF_3$, $NO_2$, CN, substituiert sein können
oder $R^1$ bildet gemeinsam mit $R^2$ den Ring

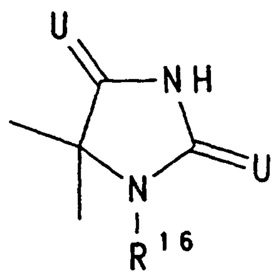

$R^2$: O-$C_1$-$C_{10}$-Alkyl$(R^{11})_n$, (n = 0, 1, 2), wobei der Alkylteil unverzweigt, verzweigt oder cyclisch ist, und $R^{11}$ mit $R^{12}$ substituiert sein kann und bei n = 2 die beiden Reste $R^{11}$ gleich oder verschieden sind.
O-$C_3$-$C_{10}$-Alkenyl$(R^{11})_n$, (n = 0, 1, 2), wobei der Alkenylteil unverzweigt, verzweigt oder cyclisch ist, sowie ein- oder mehrfach ungesättigt ist, und $R^{11}$ mit $R^{12}$ substituiert sein kann und bei n = 2 die beiden Reste $R^{11}$ gleich oder verschieden sind,
O-$C_3$-$C_{10}$-Alkinyl$(R^{11})_n$, (n = 0, 1, 2), wobei der Alkinylteil unverzweigt, verzweigt oder cyclisch ist, ein- oder mehrfach ungesättigt ist und $R^{11}$ mit $R^{12}$ substituiert sein kann;

X: $(CH_2)_m$, (m = 0, 1, 2, 3, 4) CH = CH, C≡C, $CH_2OCH_2$ oder $CH_2SCH_2$;

Y: $(CH_2)_m$, (m = 0, 1, 2, 3, 4) O, S oder $NR^8$,

Z: $(CH_2)_m$, (m = 0, 1, 2, 3, 4) S, O, S-$C_1$-$C_{10}$-Alkyl, (unverzweigt oder verzweigt), CH=CH, CH=CF, CH = CCl, CH = CBr, $CH_2$-C(O), $CH_2$-CHF, $CH_2$-CHCl, $CH_2$-CHBr, $CH_2$-CHI, $C_3$-$C_{10}$-Cycloalkylen, $C_3$-$C_{10}$-Cycloalkenylen, $COOR^7$, C≡C, CH = C($C_1$-$C_4$-Alkyl) (unverzweigt oder verzweigt), CH = C(CN), CH = C$(R^{13})$ oder $NR^8$;

$R^{16}$: $C_1$-$C_{10}$-Alkyl$(R^{11})_n$, $C_3$-$C_{10}$-Alkenyl$(R^{11})_n$, $C_3$-$C_{10}$-Alkinyl$(R^{11})_n$ mit n jeweils null oder 1.

**3.** Cyclohexanol-Ester und deren physiologisch verträgliche Salze gemäß Anspruch 1 oder 2, **dadurch gekennzeichnet, daß** die Reste in Formel I folgende Bedeutungen haben:

$R^1$: $CONHSO_2R^{14}$ oder einen Rest Rest der folgenden Formeln:

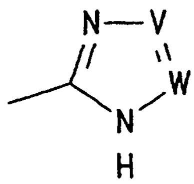 oder 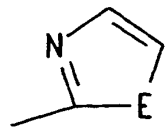

worin E = O, V = N, W = N oder $R^1$ bildet gemeinsam mit $R^2$ den Ring

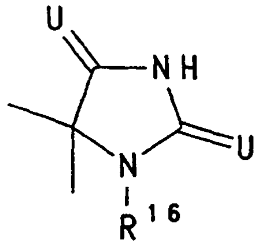

worin U Sauerstoff bedeutet,

$R^2$: O-$C_1$-$C_6$-Alkyl$(R^{11})_n$, (n = 1), wobei der Alkylteil unverzweigt, verzweigt oder cyclisch ist, O-$C_3$-$C_6$-Alkenyl $(R^{11})_n$, (n = 1), wobei der Alkenylteil unverzweigt, verzweigt oder cyclisch ist;

$R^3$, $R^{11}$ und $R^{13}$: Phenyl, mit OH oder Chlor substituiertes Phenyl, Imidazolyl, benzoanelliertes oder pyridinoanelliertes Imidazolyl, wobei $R^3$, $R^{11}$ und $R^{13}$ gleich oder verschieden sind;

$R^4$, $R^5$ und $R^6$: H oder OH, wobei $R^4$, $R^5$ und $R^6$ gleich oder verschieden sind.

$R^8$ und $R^9$: $C_1$-$C_4$-Alkyl;

$R^{14}$: $C_1$-$C_4$-Alkyl, Phenyl, Naphthyl, Thiazolyl oder dessen benzoanllierte Derivate, wobei der Aromat oder Heteroaromat mono- oder disubstituiert sein kann mit Chlor, $CF_3$, $NO_2$, $C_1$-$C_4$-Alkoxy oder $NR^8$, $R^9$, oder $R^{14}$ ist ein Rest der Formel

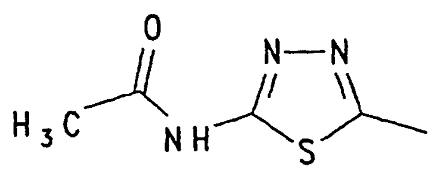

$R^{16}$: $C_1$-$C_4$-Alkyl$(R^{11})_n$ mit n = 1.

4. Verwendung von Verbindungen gemäß einem oder mehreren der Ansprüche 1 bis 3 zur Herstellung von Arzneimitteln zur Behandlung von Krankheiten, die mit einer erhöhten Aktivität des Glucose-6-Phosphatase-Systems verbunden sind.

5. Verwendung von Verbindungen gemäß einem oder mehreren der Ansprüche 1 bis 3 zur Herstellung von Arzneimitteln zur Behandlung von Krankheiten, die mit einer erhöhten Glucoseproduktion der Leber verbunden sind.

6. Verwendung von Verbindungen gemäß einem oder mehreren der Ansprüche 1 bis 3 zur Herstellung von Arzneimitteln zur Behandlung des Typs II Diabetes (nicht insulinabhängiger oder Altersdiabetes).

7. Arzneimittel enthaltend eine Verbindung nach einem oder mehreren der Ansprüche 1 bis 3.

## Claims

**1.** A cyclohexanol ester of the formula I

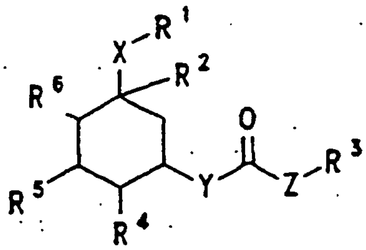

I

wherein the radicals have the following meanings:

$R^1$ is $CONHCOR^{15}$, $CSNHR^{15}$, $CONHSO_2R^{14}$, $CSNHSO_2R^{14}$ or $CH_2NHSO_2R^{14}$, or is a radical of the following formulae:

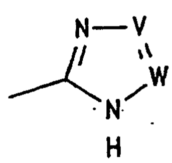
,
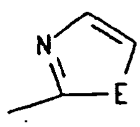
,
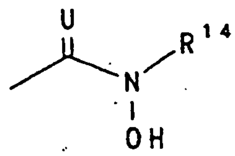
,
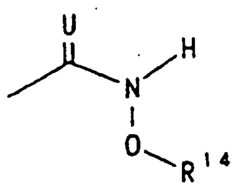
,
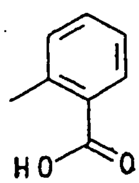
or
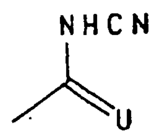

in which E is 0, S or NH, $R^{14}$ is H, U is 0 or S, V is N or CH, W is N or CH, and aromatic rings can be substituted once or more than once by F, Cl, Br, I, OH, O-$C_1$-$C_4$-alkyl, $C_1$-$C_4$-alkyl, $CF_3$, $NO_2$ or CN,
or $R^1$ forms, together with $R^2$, the ring

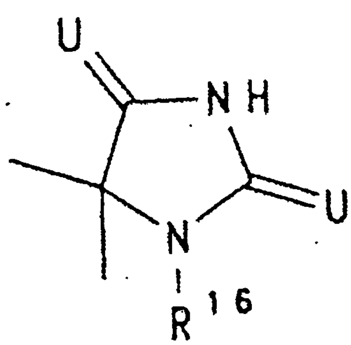

$R^2$ is $O-C_1-C_{10}$-alkyl$(R^{11})_n$, (n is 0, 1 or 2), where the alkyl moiety is unbranched, branched or cyclic, and $R^{11}$ can be substituted by $R^{12}$, and, when n is 2, the two radicals $R^{11}$ are identical or different, $O-C_3-C_{10}$-alkenyl$(R^{11})_n$, (n is 0, 1 or 2), where the alkenyl moiety is unbranched, branched or cyclic, and is unsaturated once or more than once, and $R^{11}$ can be substituted by $R^{12}$, and, when n is 2, the two radicals $R^{11}$ are identical or different,
$O-C_3-C_{10}$-alkynyl$(R^{11})_n$, (n is 0, 1 or 2), where the alkynyl moiety is unbranched, branched or cyclic, and is unsaturated once or more than once, and $R^{11}$ can be substituted by $R^{12}$,

$R^3$ $R^{11}$ and $R^{13}$ are alkyl having from 1 to 10 carbon atoms, cycloalkyl having from 3 to 8 ring carbon atoms, phenyl, naphthyl, phenanthryl, pyridyl, thienyl, furyl, pyrimidyl, indolyl, imidazolyl, coumarinyl, phthaliminyl, quinolyl, piperazinyl, tetrazolyl, triazolyl, oxazolyl or their thieno-fused, pyridino-fused, pyrimidino-fused or benzo-fused derivatives, where the aromatic radical or heteroaromatic radical can be substituted once or more than once, identically or differently, by F, Cl, Br, I, OH, $-NO_2$, CN, $C_1-C_4$-alkoxy, $C_1-C_4$-alkyl, $NR^8R^9$, phenyl, benzyl, thienyl, furyl, imidazolyl, pyridyl, 0-phenyl or 0-benzyl, and $R^3$, $R^{11}$ and $R^{13}$ are identical or different;

$R^4$, $R^5$ and $R^6$ are H, OH, an OH group protected by customary alcohol protective groups, where $R^4$, $R^5$ and $R^6$ are identical or different;

$R^8$ and $R^9$ are H, $C_1-C_4$-alkyl, $C_1-C_4$-alkanoyl or phenyl which is optionally substituted by F, Cl, Br, I, OH, $O-C_1-C_4$-alkyl, $CF_3$, $-NO_2$ or CN, where $R^8$ and $R^9$ are identical or different, or $R^8$ and $R^9$ form, together with the nitrogen atom, a 4- to 10-membered, saturated heterocyclic ring in which a $CH_2$ group can be optionally replaced by O, S or $NR^{10}$,

$R^{10}$ is H, $C_1-C_4$-alkyl, phenyl or benzyl;

$R^{12}$ is phenyl, naphthyl, phenanthryl, pyridyl, thienyl, furyl, thiazolyl, pyrimidyl, indolyl, imidazolyl, coumarinyl, phthaliminyl, quinolyl, piperazinyl, tetrazolyl, triazolyl, oxazolyl or their thieno-fused or benzo-fused derivatives, where the aromatic radical or heteroaromatic radical can be substituted once or more than once, identically or differently, by F, Cl, Br, I, OH, $CF_3$, $-NO_2$, CN, $C_1-C_4$-alkoxy, $C_1-C_4$-alkyl, $NR^8R^9$, phenyl, benzyl, thienyl, furyl, imidazolyl, pyridyl, O-phenyl or O-benzyl;

$R^{14}$ is hydrogen, $C_1-C_{10}$-alkyl, phenyl, naphthyl, phenanthryl, pyridyl, thienyl, furyl, thiazolyl, pyrimidyl, indolyl, imidazolyl, coumarinyl, phthaliminyl, quinolyl, piperazinyl, tetrazolyl, triazolyl, oxazolyl or their thieno-fused or benzo-fused derivatives, where the aromatic radical or heteroaromatic radical can be substituted once or more than once, identically or differently, by F, Cl, Br, I, OH, $CF_3$, $-NO_2$, CN, $C_1-C_4$-alkoxy, $C_1-C_4$-alkyl, $NR^8R^9$, phenyl, benzyl, thienyl, furyl, imidazolyl, pyridyl, O-phenyl or O-benzyl, or $R^{14}$ is a radical of the formula

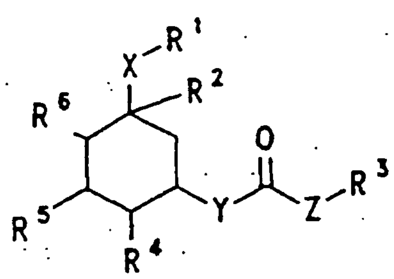

I

$R^{15}$ is $C_3-C_{10}$-alkenoyl, $C_3-C_{10}$-alkenoyl $(R^{12})$, $C_1-C_{10}$-alkanoyl$(R^{12})$, phenyl, naphthyl, phenanthryl, pyridyl, thienyl, furyl, thiazolyl, pyrimidyl, indolyl, imidazolyl, coumarinyl, phthaliminyl, quinolyl, piperazinyl, tetrazolyl, triazolyl, oxazolyl or their thieno-fused or benzo-fused derivatives, where the aromatic radical or heteroaromatic radical can be substituted once or more than once, identically or differently, by F, Cl, Br, I, OH, $CF_3$, $-NO_2$, CN, $C_1-C_4$-alkoxy, $C_1-C_4$-alkyl,

$NR^8R^9$, phenyl, benzyl, thienyl, furyl, imidazolyl, pyridyl, O-phenyl or O-benzyl;

$R^{16}$ is $C_1$-$C_{10}$-alkyl ($R^{11}$) n, $C_3$-$C_{10}$alkenyl($R^{11}$)n or $C_3$-$C_{10}$-alkynyl($R^{11}$)n, with n in each case being zero or one;

X is $(CH_2)_m$, (m is 0, 1, 2, 3 or 4) CH=CH, C=C, $CH_2OCH_2$ or $CH_2SCH_2$,

Y is $(CH_2)_m$, (m is 0, 1, 2, 3 or 4) O, S or $NR^8$;

Z is $(CH_2)_m$, (m is 0, 1, 2, 3 or 4) S, 0, S-$C_1$-$C_{10}$-alkyl, CH=CH, CH=CF, CH=CCl, CH=CBr, $CH_2$-C(O), $CH_2$-CHF, $CH_2$-CHCl, $CH_2$-CHBr, $CH_2$-CHI, $C_3$-$C_{10}$-cycloalkylene, $C_3$-$C_{10}$-cycloalkenylene, $COOR^7$, C=C, CH=C($C_1$-$C_4$-alkyl), CH=C(CN), CH=C($R^{13}$) or $NR^8$.

**2.** A cyclohexanol ester and its physiologically tolerated salts as claimed in claim 1, wherein the radicals in formula I have the following meanings:

$R^1$ is $CONHCOR^{15}$, $CSNHR^{15}$, $CONHSO_2R^{14}$, $CSNHSO_2R^{14}$ or a radical of the formulae:

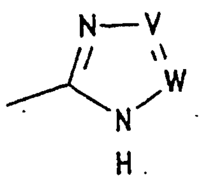 , 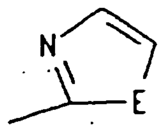 , 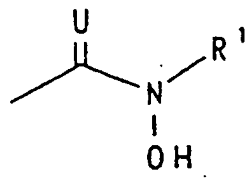 or 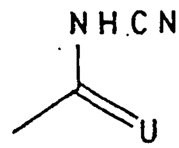

in which E is O, S or NH, $R^{14}$ is H, U is O or S, V is N or CH, W is N or CH, and aromatic rings can be substituted once or more than once by F, Cl, Br, I, OH, O-$C_1$-$C_4$-alkyl, $C_1$-$C_4$-alkyl, $CF_3$, $NO_2$ or CN, or $R^1$ forms, together with $R^2$, the ring

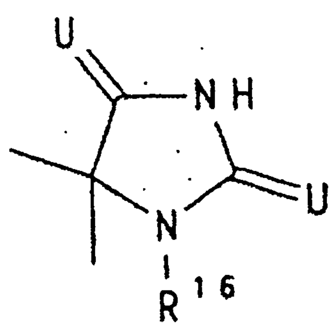

$R^2$ is O-$C_1$-$C_{10}$-alkyl$(R^{11})_n$, (n is 0, 1 or 2), where the alkyl moiety is unbranched, branched or cyclic, and $R^{11}$ can be substituted by $R^{12}$, and, when n is 2, the two radicals $R^{11}$ are identical or different,
O-$C_3$-$C_{10}$-alkenyl$(R^{11})_n$, (n is 0, 1 or 2), where the alkenyl moiety is unbranched, branched or cyclic, and is also unsaturated once or more than once, and $R^{11}$ can be substituted by $R^{12}$, and, when n is 2, the two radicals $R^{11}$ are identical or different,
O-$C_3$-$C_{10}$-alkynyl$(R^{11})_n$, (n is 0, 1 or 2), where the alkynyl moiety is unbranched, branched or cyclic, is unsaturated once or more than once, and $R^{11}$ can be substituted by $R^{12}$;

X is $(CH_2)_m$, (m is 0, 1, 2, 3 or 4) CH=CH, C≡C, $CH_2OCH_2$ or $CH_2SCH_2$;

Y is $(CH_2)_m$, (m is 0, 1, 2, 3 or 4) O, S or $NR^8$;

Z is $(CH_2)_m$, (m is 0, 1, 2, 3 or 4) S, 0, S-$C_1$-$C_{10}$-alkyl, (unbranched or branched), CH=CH, CH=CF, CH=CCl, CH=CBr, $CH_2$-C(O), $CH_2$-CHF, $CH_2$-CHCl, $CH_2$-CHBr, $CH_2$-CHI, $C_3$-$C_{10}$-cycloalkylene, $C_3$-$C_{10}$-cycloalkenylene, $COOR^7$, C=C, CH=C ($C_1$-$C_4$-alkyl) (unbranched or branched), CH=C(CN), CH=C($R^{13}$) or $NR^8$;

$R^{16}$ is $C_1$-$C_{10}$-alkyl$(R^{11})_n$, $C_3$-$C_{10}$-alkenyl$(R^{11})_n$ or $C_3$-$C_{10-}$ alkynyl$(R^{11})_n$, with n in each case being zero or 1;

**3.** A cyclohexanol ester and its physiologically tolerated salts as claimed in claim 1 or 2, wherein the radicals in formula I have the following meanings:

$R^1$ is $CONHSO_2R^{14}$ or a radical radical of the following formulae:

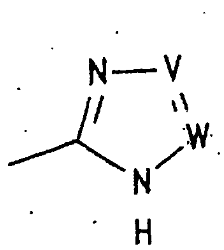

or

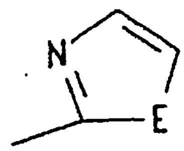

in which E is O, V is N and W is N, or $R^1$ forms, together with $R^2$, the ring

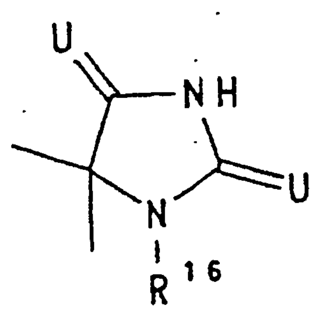

in which U is oxygen,

| | |
|---|---|
| $R^2$ | is O-$C_1$-$C_6$-alkyl$(R^{11})_n$, (n is 1), where the alkyl moiety is unbranched, branched or cyclic, or O-$C_3$-$C_6$-alkenyl$(R^{11})_n$, (n is 1), where the alkenyl moiety is unbranched, branched or cyclic; |
| $R^3$, $R^{11}$ and $R^{13}$ | are phenyl, phenyl substituted by OH or chlorine, imidazolyl or benzo-fused or pyridino-fused imidazolyl, where $R^3$, $R^{11}$ and $R^{13}$ are identical or different; |
| $R^4$, $R^5$ and $R^6$ | are H or OH, where $R^4$, $R^5$ and $R^6$ are identical or different; |
| $R^8$ and $R^9$ | are $C_1$-$C_4$-alkyl; |
| $R^{14}$ | is $C_1$-$C_4$-alkyl, phenyl, naphthyl, thiazolyl or its benzo-fused derivatives, where the aromatic radical or heteroaromatic radical can be monosubstituted or disubstituted by chlorine, $CF_3$, $NO_2$, $C_1$-$C_4$-alkoxy or $NR^8$, $R^9$, or $R^{14}$ is a radical of the formula |

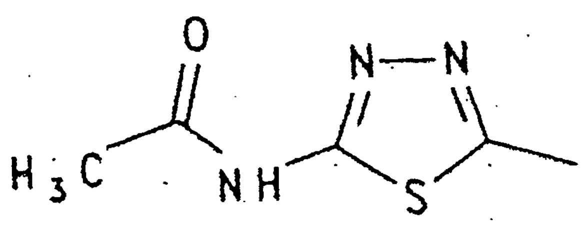

$R^{16}$ is $C_1$-$C_4$-alkyl $(R^{11})_n$, with n being 1.

**4.** The use of a compound as claimed in one or more of claims 1 to 3 for preparing pharmaceuticals for treating

diseases which are associated with an elevated activity of the glucose-6-phosphatase system.

**5.** The use of a compound as claimed in one or more of claims 1 to 3 for preparing pharmaceuticals for treating diseases which are associated with an elevated production of glucose in the liver.

**6.** The use of a compound as claimed in one or more of claims 1 to 3 for preparing pharmaceuticals for treating type II diabetes (non-insulin-dependent or maturity-onset diabetes).

**7.** A pharmaceutical containing a compound as claimed in one or more of claims 1 to 3.

**Revendications**

**1.** Esters de cyclohexanol de formule I

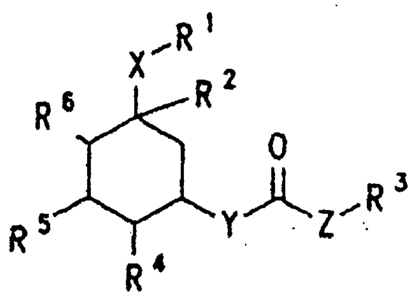

I

dans laquelle les radicaux ont les significations suivantes :

$R^1$ : $CONHCOR^{15}$, $CSNHR^{15}$, $CONHSO_2R^{14}$, $CSNHSO_2R^{14}$ ou $CH_2NHSO_2R^{14}$ ou un radical de formules suivantes :

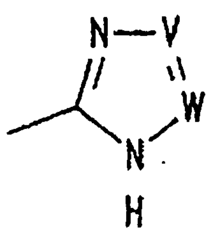 , 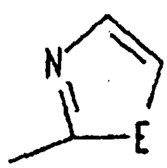 , 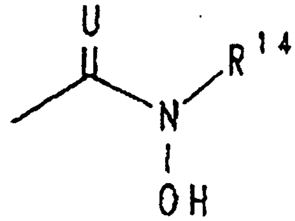 ,

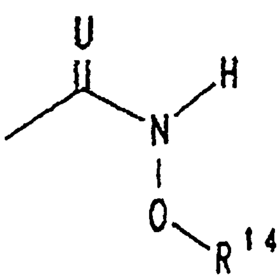 , 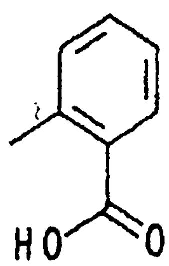 ou 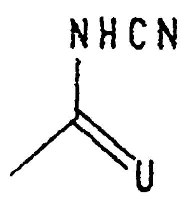

dans lesquelles E représente 0, S ou NH, $R^{14}$ représente H, U représente 0 ou S, V représente N ou CH, W représente N ou CH et les cycles aromatiques peuvent être une ou plusieurs fois substitués par F, Cl, Br, I, OH, O-alkyle en $C_1$-$C_4$, alkyle en $C_1$-$C_4$, $CF_3$, $NO_2$, CN,

ou $R^1$ forme conjointement avec $R^2$ le cycle

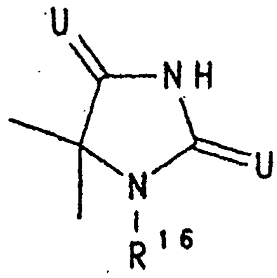

$R^2$ : O-alkyl($C_1$-$C_{10}$)-$(R^{11})_n$ (n = 0, 1, 2), le fragment alkyle étant ramifié, non ramifié ou cyclique, et $R^{11}$ pouvant être substitué par $R^{12}$, et lorsque n = 2, les deux radicaux $R^{11}$ étant identiques ou différents, O-alcényl($C_3$-$C_{10}$) -$(R^{11})_n$ (n = 0, 1, 2), le fragment alcényle étant ramifié, non ramifié ou cyclique et étant une ou plusieurs fois insaturé, et $R^{11}$ pouvant être substitué par $R^{12}$, et lorsque n = 2, les deux radicaux $R^{11}$ étant identiques ou différents,
O-alcynyl($C_3$-$C_{10}$)-$(R^{11})_n$ (n = 0, 1, 2), le fragment alcynyle étant ramifié, non ramifié ou cyclique et étant une ou plusieurs fois insaturé, et $R^{11}$ pouvant être substitué par $R^{12}$,

$R^3$, $R^{11}$ et $R^{13}$ : alkyle ayant de 1 à 10 atomes de carbone, cycloalkyle comportant 3 à 8 atomes de carbone dans le cycle, phényle, naphtyle, phénanthryle, pyridyle, thiényle, furyle, pyrimidyle, indolyle, imidazolyle, coumarinyle, phtaliminyle, quinolyle, pipérazinyle, tétrazolyle, triazolyle, oxazolyle ou leurs dérivés condensés avec un groupe thiéno, pyridino, pyrimidino ou un noyau benzénique, le cycle aromatique ou hétéroaromatique pouvant porter un ou plusieurs substituants identiques ou différents F, Cl, Br, I, OH, -$NO_2$, CN, alcoxy en $C_1$-$C_4$, alkyle en $C_1$-$C_4$, $NR^8R^9$, phényle, benzyle, thiényle, furyle, imidazolyle, pyridyle, O-phényle ou O-benzyle, et $R^3$, $R^{11}$ et $R^{13}$ étant identiques ou différents ;

$R^4$, $R^5$ et $R^6$ : H, OH, un groupe OH protégé par des groupes protecteurs de fonction alcool usuels, $R^4$, $R^5$ et $R^6$ étant identiques ou différents ;

$R^8$ et $R^9$ : H, alkyle en $C_1$-$C_4$, alcanoyle en $C_1$-$C_4$, phényle qui est éventuellement substitué par F, Cl, Br, I, OH, O-alkyle en $C_1$-$C_4$, $CF_3$, -$NO_2$ ou CN, $R^8$ et $R^9$ étant identiques ou différents, ou $R^8$ et $R^9$ forment conjointement avec l'atome d'azote un cycle hétérocyclique saturé à 4-10 chaînons, dans lequel un groupe $CH_2$ peut éventuellement être remplacé par O, S ou $NR^{10}$,

$R^{10}$ : H, alkyle en $C_1$-$C_4$, phényle ou benzyle ;

$R^{12}$ : phényle, naphtyle, phénanthryle, pyridyle, thiényle, furyle, thiazolyle, pyrimidyle, indolyle, imidazolyle, coumarinyle, phtaliminyle, quinolyle, pipérazinyle, tétrazolyle, triazolyle, oxazolyle ou leurs dérivés condensés avec un groupe thiéno ou un noyau benzénique, le cycle aromatique ou hétéroaromatique pouvant porter un ou plusieurs substituants identiques ou différents F, Cl, Br, I, OH, $CF_3$, -$NO_2$, CN, alcoxy en $C_1$-$C_4$, alkyle en $C_1$-$C_4$, $NR^8R^9$, phényle, benzyle, thiényle, furyle, imidazolyle, pyridyle, O-phényle ou O-benzyle ;

$R^{14}$ : hydrogène, alkyle en $C_1$-$C_{10}$, phényle, naphtyle, phénanthryle, pyridyle, thiényle, furyle, thiazolyle, pyrimidyle, indolyle, imidazolyle, coumarinyle, phtaliminyle, quinolyle, pipérazinyle, tétrazolyle, triazolyle, oxazolyle ou leurs dérivés condensés avec un groupe thiéno ou un noyau benzénique, le cycle aromatique ou hétéroaromatique pouvant porter un ou plusieurs substituants identiques ou différents F, Cl, Br, I, OH, $CF_3$, -$NO_2$, CN, alcoxy en $C_1$-$C_4$, alkyle en $C_1$-$C_4$, $NR^8R^9$, phényle, benzyle, thiényle, furyle, imidazolyle, pyridyle, O-phényle ou O-benzyle, ou $R^{14}$ est un radical de formule

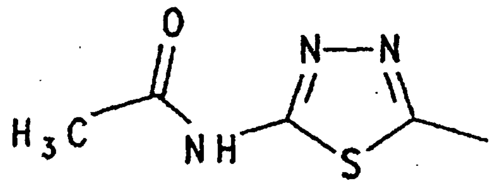

$R^{15}$ : alcénoyle en $C_3$-$C_{10}$, alcénoyl($C_3$-$C_{10}$)-($R^{12}$), alcanoyl ($C_1$-$C_{10}$)-($R^{12}$), phényle, naphtyle, phénanthryle, pyridyle, thiényle, furyle, thiazolyle, pyrimidyle, indolyle, imidazolyle, coumarinyle, phtaliminyle, quinolyle, pipérazinyle, tétrazolyle, triazolyle, oxazolyle ou leurs dérivés condensés avec un groupe thiéno ou un noyau benzénique, le cycle aromatique ou hétéroaromatique pouvant porter un ou plusieurs substituants identiques ou différents F, Cl, Br, I, OH, $CF_3$, $-NO_2$, CN, alcoxy en $C_1$-$C_4$, alkyle en $C_1$-$C_4$, $NR^8R^9$, phényle, benzyle, thiényle, furyle, imidazolyle, pyridyle, O-phényle ou O-benzyle ;

$R^{16}$ : alkyl($C_1$-$C_{10}$)-($R^{11}$)$_n$, alcényl($C_3$-$C_{10}$)-($R^{11}$)$_n$ ou alcynyl($C_3$-$C_{10}$)($R^{11}$)$_n$, n étant chaque fois 0 ou 1 ;

X : $(CH_2)_m$ (m = 0, 1, 2, 3, 4), CH=CH, C=C, $CH_2OCH_2$ ou $CH_2SCH_2$ ;

Y : $(CH_2)_m$ (m = 0, 1, 2, 3, 4), O, S ou $NR^8$ ;

Z : $(CH_2)_m$ (m = 0, 1, 2, 3, 4), S, O, S-alkyl-($C_1$-$C_{10}$), CH=CH, CH=CF, CH=CCl, CH=CBr, $CH_2$-C(O), $CH_2$-CHF, $CH_2$-CHCl, $CH_2$-CHBr, $CH_2$-CHI, cycloalkylène en $C_3$-$C_{10}$, cycloalcénylène en $C_3$-$C_{10}$, $COOR^7$, C≡C, CH=C (alkyle($C_1$-$C_4$)], CH=C(CN), CH=C($R^{13}$) ou $NR^8$.

2. Esters de cyclohexanol et leurs sels physiologiquement acceptables selon la revendication 1, **caractérisés en ce que** les radicaux dans la formule I ont les significations suivantes :

$R^1$ : $CONHCOR^{15}$, $CSNHR^{15}$, $CONHSO_2R^{14}$, $CSNHSO_2R^{14}$ ou un radical de formules :

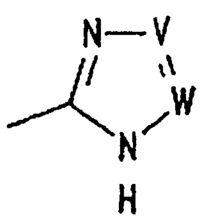 , 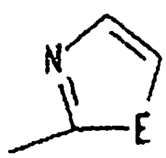 , 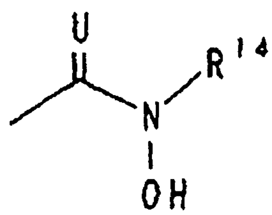 ou 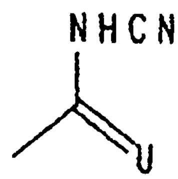

dans lesquelles E représente O, S ou NH, $R^{14}$ représente H, U représente O ou S, V représente N ou CH, W représente N ou CH et les cycles aromatiques peuvent être une ou plusieurs fois substitués par F, Cl, Br, I, OH, O-alkyle en $C_1$-$C_4$, alkyle en $C_1$-$C_4$, $CF_3$, $NO_2$, CN,
ou $R^1$ forme conjointement avec $R^2$ le cycle

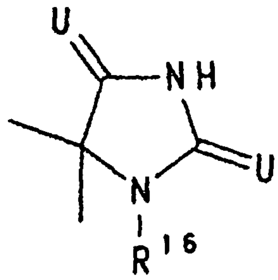

$R^2$ : O-alkyl($C_1$-$C_{10}$)-($R^{11}$)$_n$ (n = 0, 1, 2), le fragment alkyle étant ramifié, non ramifié ou cyclique, et $R^{11}$ pouvant être substitué par $R^{12}$, et lorsque n = 2, les deux radicaux $R^{11}$ étant identiques ou différents,
O-alcényl($C_3$-$C_{10}$)-($R^{11}$)$_n$(n = 0, 1, 2), le fragment alcényle étant ramifié, non ramifié ou cyclique et étant une ou plusieurs fois insaturé, et $R^{11}$ pouvant être substitué par $R^{12}$, et lorsque n = 2, les deux radicaux $R^{11}$ étant identiques ou différents,
O-alcynyl($C_3$-$C_{10}$)-($R^{11}$)$_n$ (n = 0, 1, 2), le fragment alcynyle étant ramifié, non ramifié ou cyclique et étant une ou plusieurs fois insaturé, et $R^{11}$ pouvant être substitué par $R^{12}$,

X : $(CH_2)_m$ (m = 0, 1, 2, 3, 4), CH=CH, C≡C, $CH_2OCH_2$ ou $CH_2SCH_2$ ;

Y : $(CH_2)_m$ (m = 0, 1, 2, 3, 4), 0, S ou $NR^8$ ;

Z : $(CH_2)_m$ (m = 0, 1, 2, 3, 4), S, O, S-alkyle-($C_1$-$C_{10}$) (ramifié ou non ramifié), CH=CH, CH=CF, CH=CCl, CH=CBr, $CH_2$-C(O), $CH_2$-CHF, $CH_2$-CHCl, $CH_2$-CHBr, $CH_2$-CHI, cycloalkylène en $C_3$-$C_{10}$, cycloalcénylène en $C_3$-$C_{10}$, $COOR^7$, C≡C, CH=C[alkyle($C_1$-$C_4$)] (ramifié ou non ramifié), CH=C(CN), CH=C($R^{13}$) ou $NR^8$ ;

$R^{16}$ : alkyl($C_1$-$C_{10}$)-($R^{11}$)$_n$, alcényl ($C_3$-$C_{10}$)-($R^{11}$)$_n$, alcynyl($C_3$-$C_{10}$)-($R^{11}$)$_n$, n étant chaque fois 0 ou 1.

**3.** Esters de cyclohexanol et leurs sels physiologiquement acceptables selon la revendication 1 ou 2, **caractérisés en ce que** les radicaux dans la formule I ont les significations suivantes :

$R^1$ : $CONHSO_2R^{14}$ ou un radical de formules suivantes :

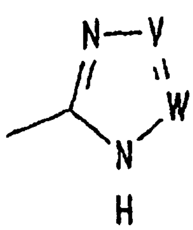

ou

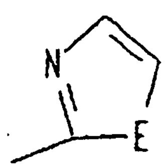

dans lesquelles E représente 0, V représente N, W représente N ou $R^1$ forme conjointement avec $R^2$ le cycle

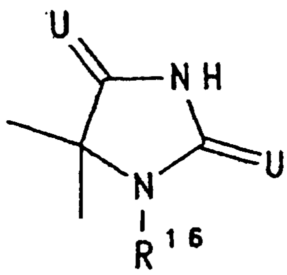

où U représente un atome d'hydrogène,

$R^2$ : O-alkyl ($C_1$-$C_6$)-($R^{11}$)$_n$ (n = 1), le fragment alkyle étant ramifié, non ramifié ou cyclique, O-alcényl($C_3$-$C_6$) -($R^{11}$)$_n$ (n = 1), le fragment alcényle étant ramifié, non ramifié ou cyclique ;

$R^3$, $R^{11}$ et $R^{13}$ : phényle, phényle substitué par OH ou un atome de chlore, imidazolyle, imidazolyle condensé avec un noyau benzénique ou condensé avec un groupe pyridino, $R^3$, $R^{11}$ et $R^{13}$ étant identiques ou différents ;

$R^4$, $R^5$ et $R^6$ : H ou OH, $R^4$, $R^5$ et $R^6$ étant identiques ou différents ;

$R^8$ et $R^9$ : alkyle en $C_1$-$C_4$ ;

$R^{14}$ : alkyle en $C_1$-$C_4$, phényle, naphtyle, thiazolyle ou leurs dérivés condensés avec un noyau benzénique, le cycle aromatique ou hétéroaromatique pouvant être mono- ou disubstitué par un ou des atomes de chlore ou groupes $CF_3$, $NO_2$, alcoxy en $C_1$-$C_4$ ou $NR^8$, $R^9$, ou bien $R^{14}$ est un radical de formule

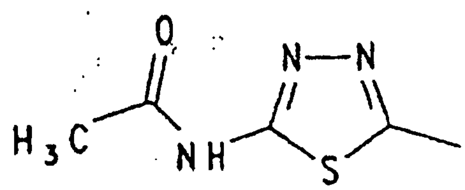

$R^{16}$ : alkyl($C_1$-$C_4$)-($R^{11}$)$_n$ où n = 1.

**4.** Utilisation de composés selon une ou plusieurs des revendications 1 à 3, pour la fabrication de médicaments destinés au traitement de maladies qui sont liées à une activité augmentée du système glucose-6-phosphatase.

**5.** Utilisation de composés selon une ou plusieurs des revendications 1 à 3, pour la fabrication de médicaments destinés au traitement de maladies qui sont liées à une production augmentée de glucose dans le foie.

**6.** Utilisation de composés selon une ou plusieurs des revendications 1 à 3, pour la fabrication de médicaments destinés au traitement du diabète de type II (diabète non insulino-dépendant ou diabète de la maturité).

**7.** Médicament contenant un composé selon une ou plusieurs des revendications 1 à 3.